# EUROPEAN PATENT APPLICATION

(11) **EP 4 566 604 A1**
(43) Date of publication of application: **11.06.2025**
(21) Application number: 23850462.5
(22) Date of filing: 03.08.2023
(51) Int. Cl.: A61K 31/405, A61P 25/28, A23L 33/10

(54) **USE OF NOVEL PPAR ACTIVITY MODULATOR FOR PREVENTION, AMELIORATION OR TREATMENT OF DEGENERATIVE BRAIN DISEASES**

(30) Priority: 03.08.2022 KR 20220096513
(71) Applicant: POSTECH Research and Business Development Foundation, Pohang-si, Gyeongsangbuk-do 37673 (KR); Novmetapharma Co., Ltd., Seoul 06050 (KR)
(72) Inventor: KIM, Kyongtai, Pohang-si, Gyeongsangbuk-do 37673 (KR); KANG, Jeonghwa, Pohang-si, Gyeongsangbuk-do 37673 (KR); OH, Eunji, Pohang-si, Gyeongsangbuk-do 37673 (KR); JUNG, Hoe Yune, Pohang-si, Gyeongsangbuk-do 37668 (KR); LEE, Heon Jong, Incheon 21048 (KR)
(74) Representative: Maiwald GmbH
(86) International application number: PCT/KR2023/011420
(87) International publication number: WO 2024/029962

(57) **Abstract**

The present invention relates to a use of a novel PPAR activity modulator for the prevention, amelioration or treatment of degenerative brain diseases and, more specifically, provides a composition for the prevention, amelioration or treatment of degenerative brain diseases by using a PPAR active agent that binds to and activates all subtypes of PPAR of PPAR, and a composition for enhancing learning ability, improving memory, or ameliorating cognitive decline. Also, the present invention provides a method for the prevention, amelioration or treatment of degenerative brain diseases by using the novel PPAR active agent, and a method for enhancing learning ability, improving memory or ameliorating cognitive decline.

## Description

### [Technical Field]

The present invention relates to a use of a novel PPAR activity modulator for the prevention, amelioration or treatment of degenerative brain diseases and, more specifically, provides a composition for the prevention, amelioration or treatment of degenerative brain diseases by using a PPAR active agent that binds to and activates all subtypes of PPAR of PPAR**,** and a composition for enhancing learning ability, improving memory, or ameliorating cognitive decline. Also, the present invention provides a method for the prevention, amelioration or treatment of degenerative brain diseases by using the novel PPAR active agent, and a method for enhancing learning ability, improving memory or ameliorating cognitive decline.

### [Background Art]

The rapid modernization has led to the rise of the income level of people and the development of a medical and health environment of society, thereby a proportion of the elderly population is gradually increasing worldwide. The growth of the elderly population has resulted in an increase in the prevalence of age-related diseases, most notably senile dementia, a degenerative brain disorder, and the socioeconomic cost caused by the increase in the number of dementia patients has also increased.

Many people with senile dementia have Alzheimer's disease, but there are currently no effective treatments or prevention for the disease. Recently, Aducanumab and Lecanemab, antibody therapeutics targeting amyloid-beta aggregates for the treatment of Alzheimer's dementia, have been approved by the U.S. Food and Drug Administration (FDA), but issues regarding the validity of their therapeutic efficacy remain unresolved and other drugs prescribed for Alzheimer's dementia include five types, such as acetylcholine esterase inhibitors and glutamate receptor inhibitors, but all of them are limited to alleviating symptoms.

Degenerative brain diseases, including Alzheimer's dementia, have a common characteristic of abnormal protein aggregate formation in brain tissue, which leads to chronic inflammation. In the case of Alzheimer's dementia, Aβ peptides aggregate to form plaques outside the cells, leading to the production of reactive oxygen species and triggering inflammatory reactions caused by microglia. During this process, excessively activated microglia secrete cytokines (IL-6, TNFα) and reactive oxygen species (ROS), which promote the apoptosis of surrounding neurons. As it has been reported through many papers that the action of microglia causes the generation and severe pathology of protein aggregates and the death of neurons, the development of therapeutic agents for reducing the chronic inflammatory action of brain tissues along with the direction of reducing protein aggregates is actively progressing.

In this regard, Korean Patent Laid-Open No. 10-2022-0060494 discloses that chlorpromazine regulates microglial activity to alleviate neuroinflammation, thereby exhibiting therapeutic effects on degenerative brain diseases, and Korean Patent Laid-Open No. 10-2022-0039607 discloses that N-palmitoyl serinol protects the brain by reducing the amounts of inflammatory cytokines in brain tissue, thereby showing effectiveness in improving cognitive ability and memory.

Peroxisome proliferator-activated receptor (PPAR) is a ligand-mediated transcription factor and is primarily known as a key regulator of metabolic processes. PPAR exists in three subtypes: PPARα, PPARβ/δ, and PPARγ, and antagonists targeting these are used as therapeutics for conditions such as dyslipidemia, diabetes, and obesity. PPAR has been reported to be closely associated with inflammatory responses, as existing studies have revealed that it regulates the quantitative changes or activity levels of NF-κB, a key regulator of inflammatory responses, in addition to its role in metabolic processes. According to a 2021 clinical trial report on Alzheimer's dementia therapeutics, Losartan, a PPARγ antagonist, is undergoing Phase 3 clinical trials, and various studies have shown that a pan-agonist capable of activating all three PPAR subtypes also exhibits memory-enhancing effects in animal models of Alzheimer's dementia.

Against this background, the present inventors screened novel synthetic small-molecule compounds capable of regulating PPAR activity and confirmed that the screened compounds modulate inflammatory responses in microglia and exhibit memory-enhancing effects when administered to animal models of Alzheimer's dementia, thereby discovering the potential of these compounds for the prevention, amelioration, and treatment of pathological conditions associated with Alzheimer's dementia, and for the enhancement of learning ability, the improvement of memory or the amelioration of cognitive decline, and completing the present invention.

### [Disclosure]

### [Technical Problem]

Accordingly, an object of the present invention is to provide a composition for preventing, ameliorating, or treating degenerative brain diseases including a novel PPAR active agent that exhibits the effect of inhibiting excessive inflammatory responses through the activation of PPAR transcription factors in microglia.

Another object of the present invention is to provide a composition for enhancing learning ability, improving memory or ameliorating cognitive decline, including the aforementioned novel PPAR active agent.

Still another object of the present invention is to provide a method for preventing, ameliorating, or treating degenerative brain diseases including the aforementioned novel PPAR active agent.

Still another object of the present invention is to provide a method for enhancing learning ability, improving memory or ameliorating cognitive decline including the aforementioned novel PPAR active agent.

Still another object of the present invention is to provide a use of the aforementioned novel PPAR active agent for the manufacture of a medicament for preventing, ameliorating, or treating degenerative brain diseases.

Still another aim of the present invention is to provide a use of the aforementioned novel PPAR active agent for the manufacture of a medicament for enhancing learning ability, improving memory, or ameliorating cognitive decline.

However, the technical challenges intended to be addressed by the present invention are not limited to those mentioned above, and other challenges not explicitly mentioned will be clearly understood by those skilled in the art from the following description.

### [Technical Solution]

To solve the above-mentioned problems, the present invention provides a pharmaceutical composition for preventing or treating degenerative brain diseases, comprising a compound represented by Chemical Formula 1 below, a stereoisomer thereof or a pharmaceutically acceptable salt thereof as an active ingredient:

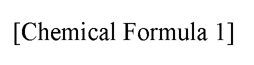

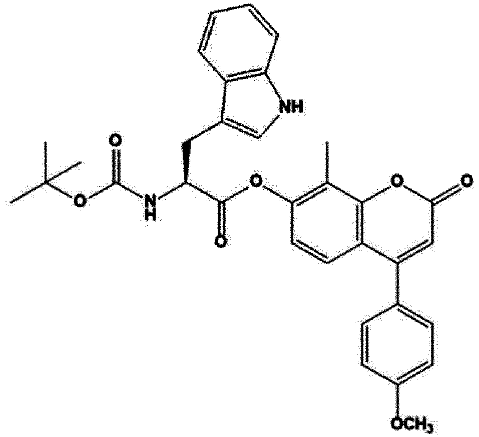

In addition, the present invention provides a health functional food composition for preventing or ameliorating degenerative brain diseases, comprising a compound represented by Chemical Formula 1 above, a stereoisomer thereof or a sitologically acceptable salt thereof as an active ingredient.

In addition, the present invention provides a composition for enhancing learning ability, improving memory or ameliorating cognitive function, comprising a compound represented by Chemical Formula 1 above, a stereoisomer thereof or a pharmaceutically or sitologically acceptable salt thereof as an active ingredient.

Additionally, the present invention provides a method for preventing, ameliorating, or treating degenerative brain diseases, comprising administering a compound represented by Chemical Formula 1 above, a stereoisomer thereof or a pharmaceutically or sitologically acceptable salt thereof to a subject in need thereof.

Additionally, the present invention provides a method for enhancing learning ability, improving memory or ameliorating cognitive decline, comprising administering a compound represented by Chemical Formula 1 above, a stereoisomer thereof or a pharmaceutically or sitologically acceptable salt thereof to a subject in need thereof.

Furthermore, the present invention provides a use of a compound represented by Chemical Formula 1 above, a stereoisomer thereof or a pharmaceutically or sitologically salt thereof for the manufacture of a medicament for preventing, ameliorating, or treating degenerative brain diseases.

Furthermore, the present invention provides a use of a compound represented by Chemical Formula 1 above, stereoisomers thereof or pharmaceutically or sitologically salts thereof for the manufacture of a medicament for enhancing learning ability, improving memory or ameliorating cognitive decline.

In addition, the stereoisomers may comprise a racemate, an enantiomer, a diastereomer, a mixture of enantiomers or a mixture of diastereomers.

In addition, the degenerative brain diseases may comprise one or more selected from the group consisting of Dementia, Autism, Alzheimer's disease, Huntington's disease, Vascular dementia, Stroke, Ischemic stroke, Traumatic brain injury, Amnesia, Parkinson's disease, Pick disease, Creutzfeldt-Jakob disease, Peripheral neuropathy, Amyotrophic lateral sclerosis, multiple sclerosis and mild cognitive impairment.

In addition, the compound, the stereoisomer thereof or the sitologically salt thereof may activate all of PPARα, PPARβ/δ and PPARγ.

In addition, the compound, the stereoisomer thereof or the pharmaceutically or sitologically salt thereof may exhibit one or more activities selected from the following i) to iii):
i) inhibiting inflammatory responses by activating PPAR transcription factors in microglia;
ii) inhibiting the formation of Aβ aggregates in brain tissue; and
iii) inhibiting gliosis caused by excessive activity of microglia and astrocytes.

Furthermore, the composition for enhancing learning ability, improving memory, or ameliorating cognitive decline may be a pharmaceutical or health functional food composition.

### [Advantageous Effects]

The compound represented by Chemical Formula 1 of the present invention, as a PPAR active agent, exhibits an effect of inhibiting excessive inflammatory responses elevated in Alzheimer's dementia by activating PPAR transcription factors in microglia, thereby inhibiting the formation of Aβ aggregates known as a cause of Alzheimer's dementia. Also, it inhibits gliosis caused by microglia and astrocytes in the brain tissue of 5xFAD dementia-induced mouse and shows enhancement effect on learning ability, improvement effect on memory, and amelioration effect on cognitive decline, making it applicable as pharmaceuticals or a health functional food for the prevention, amelioration, or treatment of degenerative brain diseases and for the enhancement of learning ability, the improvement of memory, and the amelioration of cognitive decline.

### [Description of Drawings]

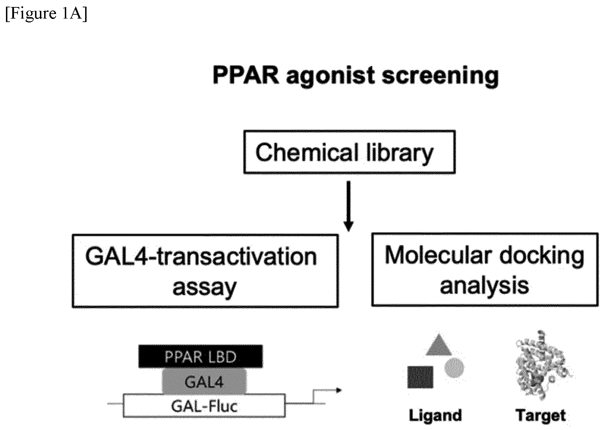

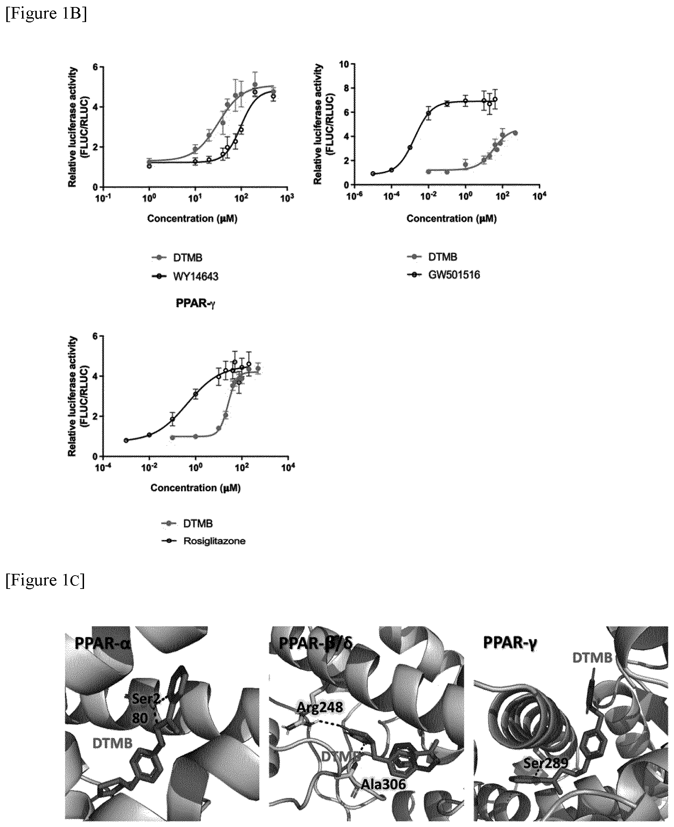

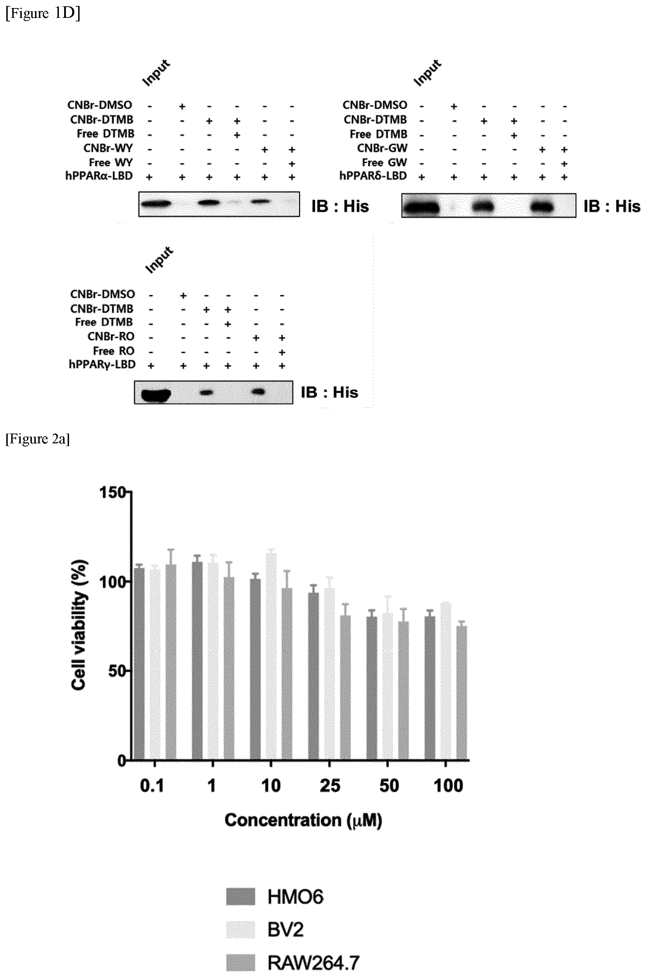

FIGS. 1A to 1D represent the comparison of PPAR transcription factor activation levels following DTMB treatment, where FIG. 1A is a schematic diagram showing the screening method for PPAR active agents, FIG. 1B shows the results of PPAR activity analysis using a GAL4 reporter vector, FIG. 1C presents the results of molecular docking analysis, and FIG. 1D displays the results of binding experiments between CNBr-drugs and proteins.
FIG. 2A represents the results of MTT analysis testing DTMB cytotoxicity in a concentration-dependent manner using HMO6, BV2, and RAW 264.7 cell lines.

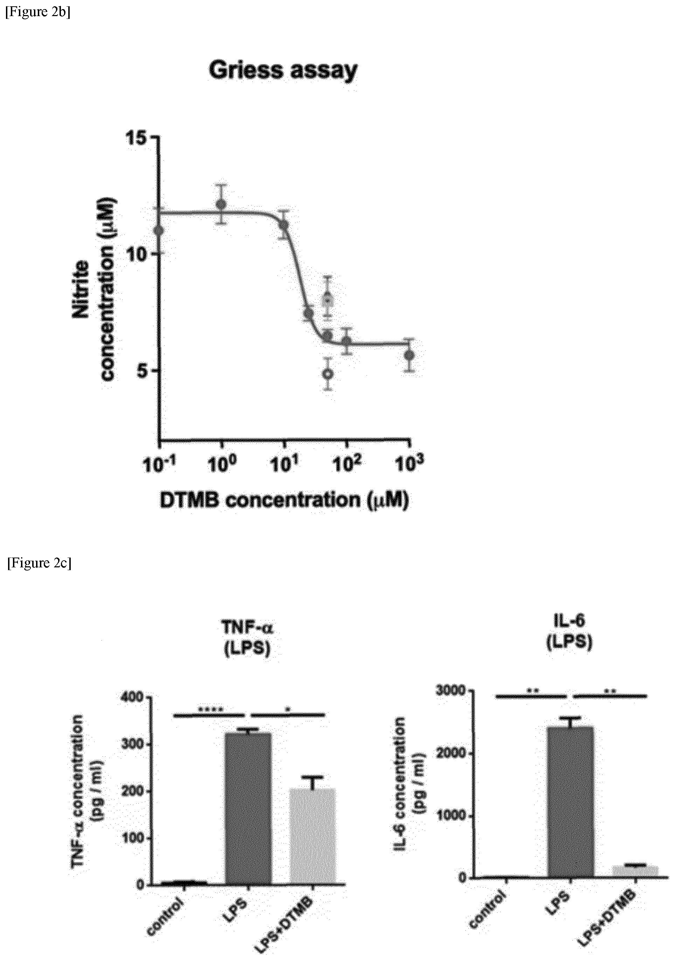

FIG. 2B represents the anti-inflammatory effects of DTMB treatment, measured by NO production, in macrophage cell lines where inflammatory responses were induced by lipopolysaccharide (LPS) treatment.

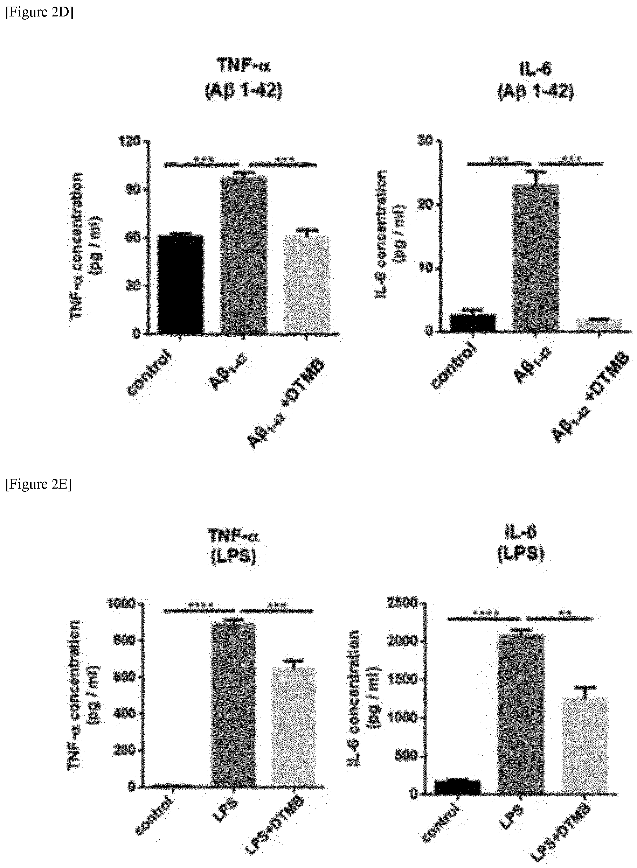

FIG. 2C and FIG. 2D represent the anti-inflammatory effects of DTMB treatment in BV2 microglial cell lines where inflammatory responses were induced by LPS and Aβ aggregates, respectively, measured by cytokine production levels. The data represent the mean ± standard error of the mean (SEM) from three independent experiments. Statistical significance was analyzed by one-way ANOVA: *p < 0.05; **p < 0.01; ***p < 0.001.

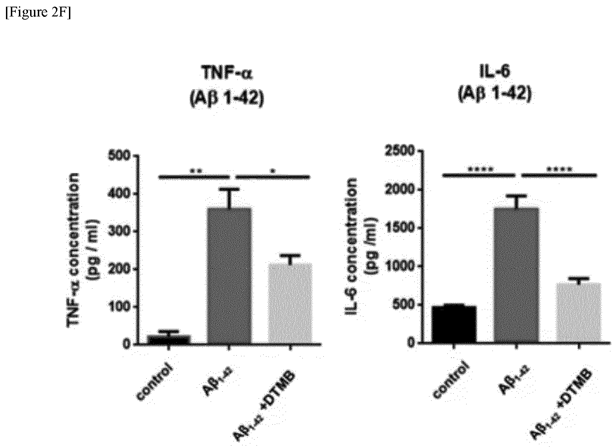

FIG. 2E and FIG. 2F represent the anti-inflammatory effects of DTMB treatment in primary microglia isolated from mouse, where inflammatory responses were induced by LPS and Aβ aggregates, respectively, measured by cytokine production levels. The quantification of blot intensities was performed using ImageJ. The data represent the mean ± standard error of the mean (SEM) from three independent experiments. Statistical significance was analyzed by one-way ANOVA: *p < 0.05; **p < 0.01; ***p < 0.001. The quantification of blot intensities was performed using ImageJ. The data represent the mean ± standard error of the mean (SEM) from three independent experiments. Statistical significance was analyzed by one-way ANOVA: *p < 0.05; **p < 0.01; ***p < 0.001.

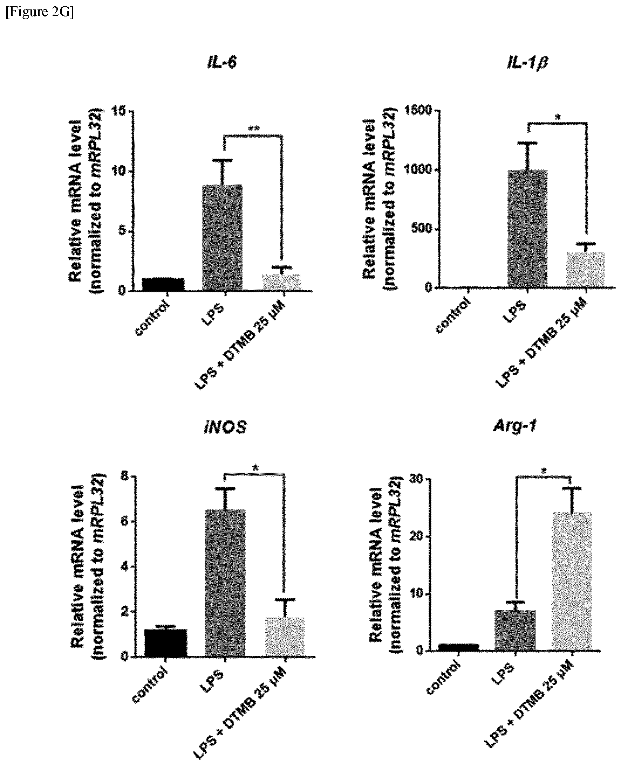

FIG. 2G represents the results of quantitative PCR analysis of mRNA expression levels of pro-inflammatory cytokines (Il-6 and Il-1β), related enzymes (iNOS), and M2 phenotype microglia markers (Arg-1). The data represent the mean ± standard error of the mean (SEM) from three independent experiments. Statistical significance was analyzed by one-way ANOVA: *p < 0.05; **p < 0.01; ***p < 0.001.

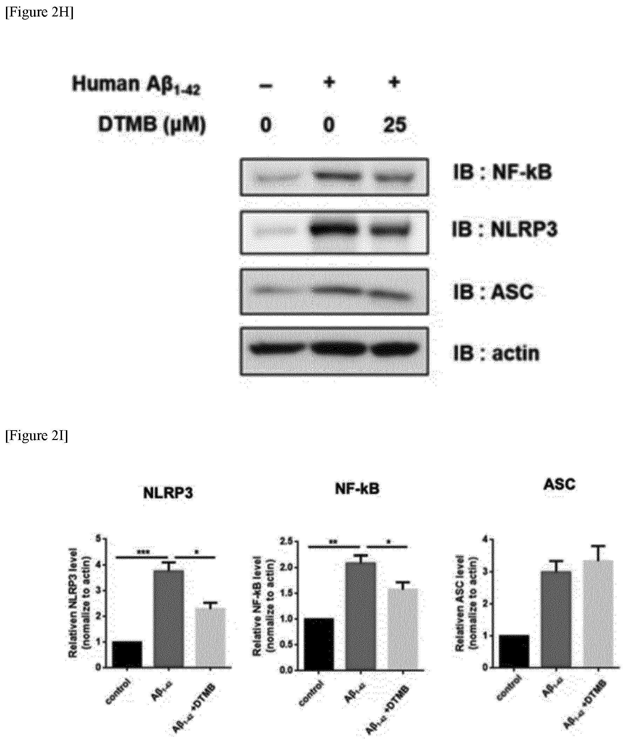

FIG. 2H represents the results of Western blotting analysis showing the reduction effects of DTMB treatment on NF-κB and inflammation-related factors (NLRP-3 and ASC) in primary microglia isolated from mouse.
FIG. 2I represents the results showing changes in the protein expression levels of NF-κB and inflammation-related factors (NLRP-3 and ASC) following DTMB treatment in primary microglia isolated from mouse. The data represent the mean ± standard error of the mean (SEM) from three independent experiments. Statistical significance was indicated by p-values analyzed using one-way ANOVA as follows: *p < 0.05; **p < 0.01; ***p < 0.001.

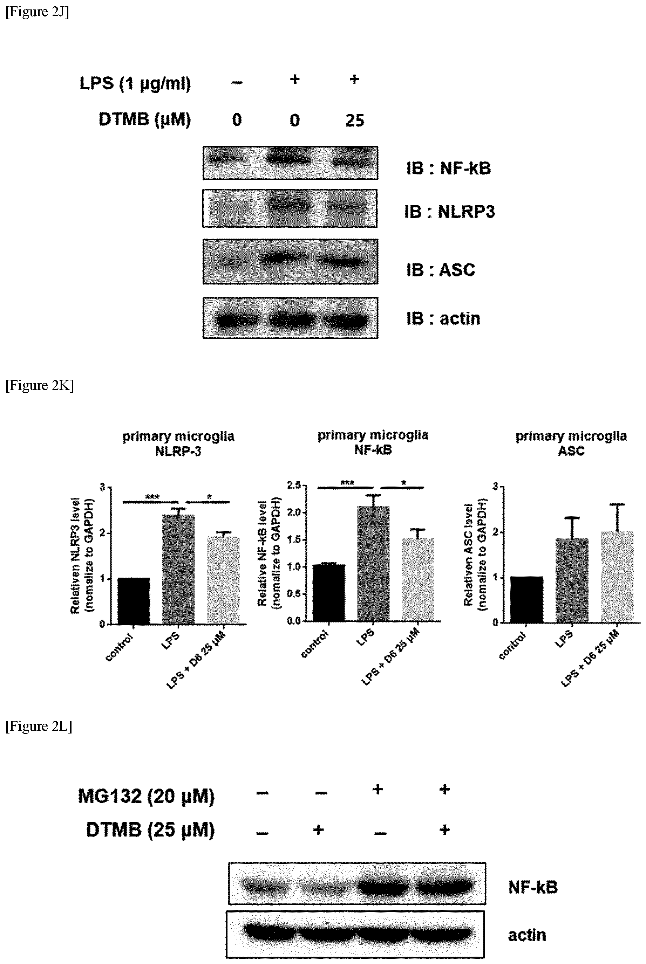

FIG. 2J and FIG. 2K represent representative Western blot data for NF-κB, NLRP3, and ASC in primary microglia treated with DMSO (0.1%) or DTMB (25 µM) for 4 hours. Inflammatory responses were induced using LPS (1 µg/mL). The data represent the mean ± standard error of the mean (SEM) from three independent experiments. Statistical significance was indicated by p-values analyzed using one-way ANOVA as follows: *p < 0.05; **p < 0.01; ***p < 0.001.

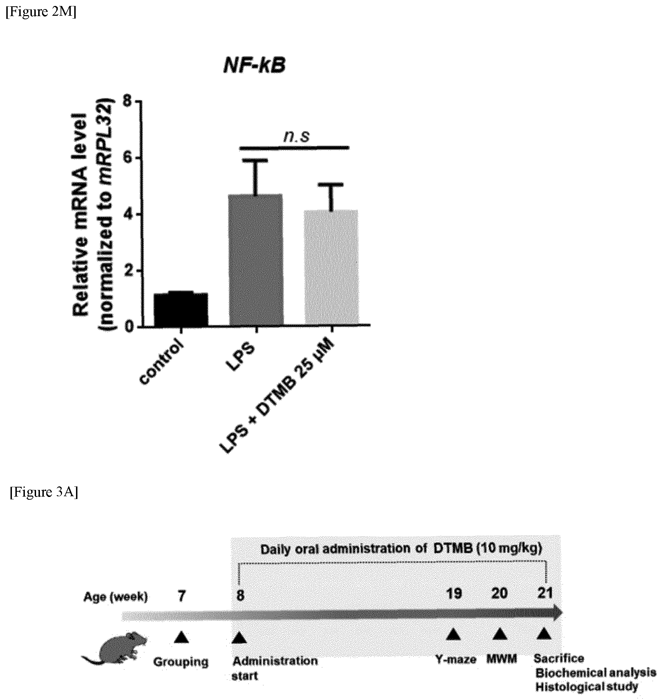

FIG. 2L and FIG. 2M represent the results of NF-κB gene expression levels following treatment of cells with MG132 (20 µM) for 6 hours to inhibit proteasomal degradation, followed by treatment with DTMB (25 µM) for 24 hours. The data represent the mean ± standard error of the mean (SEM) from three independent experiments. Statistical significance was indicated by p-values analyzed using one-way ANOVA as follows: *p < 0.05; **p < 0.01; ***p < 0.001.

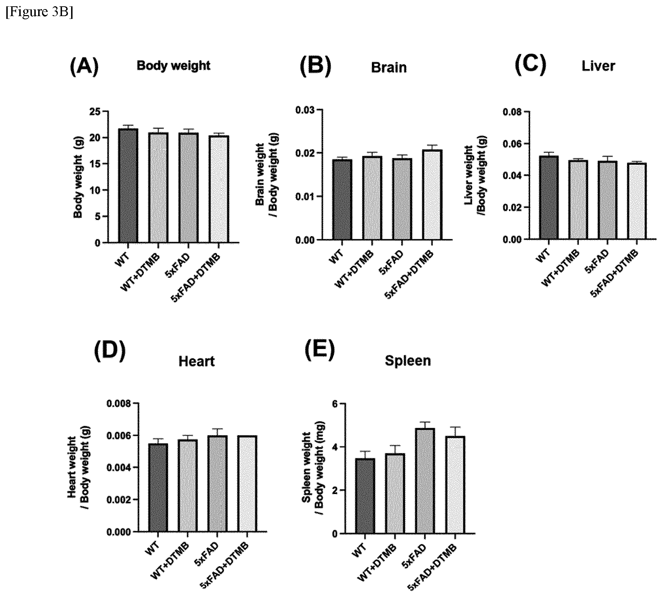

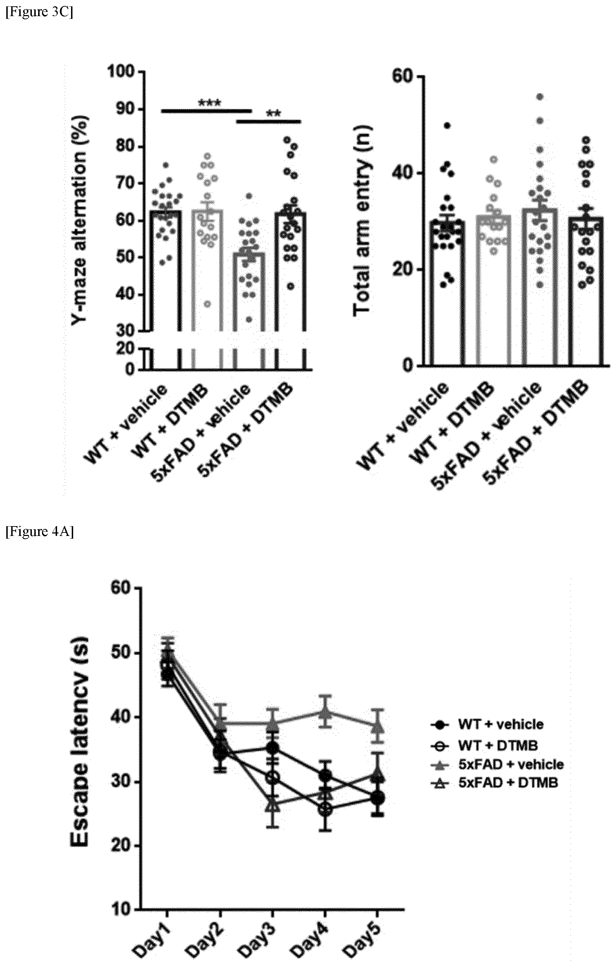

FIG. 3A represents a schematic diagram of the experimental design to evaluate the effects of DTMB in a 5xFAD Alzheimer's dementia animal model, FIG. 3B shows the changes in body weight and relative organ weight after DTMB treatment in the 5xFAD mouse model, and FIG. 3C represents the results confirming the improvement in short-term memory and cognitive function due to DTMB administration in the 5xFAD Alzheimer's dementia animal model through Y-maze test analysis. In FIG. 3C, the left graph indicates the change behavior of mouse in each group, and the right graph shows the total arm entries, representing the total number of entries into each section. All data are presented as the mean ± standard error of the mean (SEM) (n = 25 per group). p-values were indicated as follows based on two-way ANOVA: **p < 0.01, ***p < 0.001.

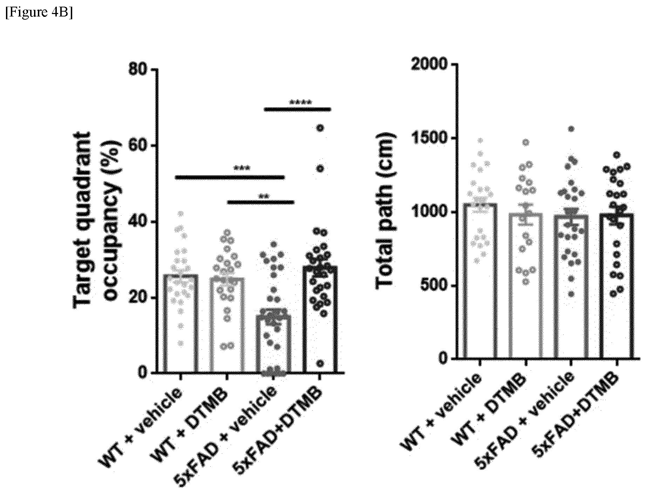

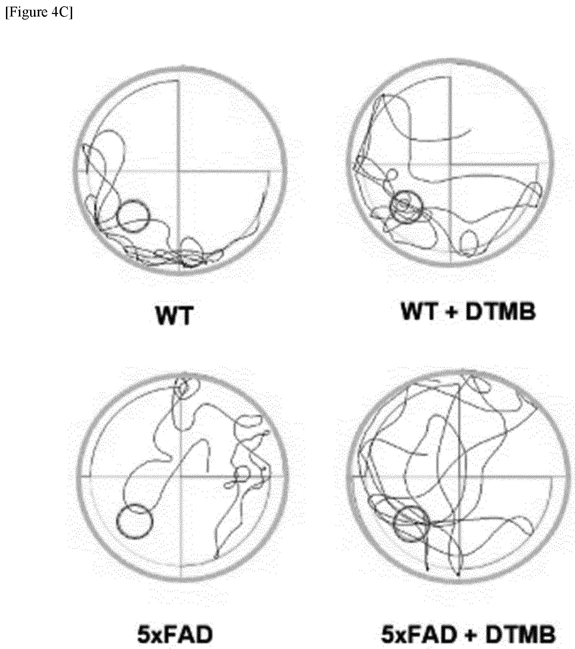

FIGS. 4A to 4C represent the results confirming the improvement in long-term memory and cognitive function due to DTMB administration in the 5xFAD Alzheimer's dementia animal model through water maze test analysis, where FIG. 4A shows the time taken (escape latency) for mouse in each group to find the platform, FIG. 4b indicates the time spent in the target quadrant (target quadrant occupancy) and the total swimming distance (total path) for each group of mouse, and FIG. 4c represents the movement trajectories of mouse in each group. All data are presented as the mean ± standard error of the mean (SEM) (n = 25 per group), and p-values were indicated as follows based on two-way ANOVA: **p < 0.01, ***p < 0.001.

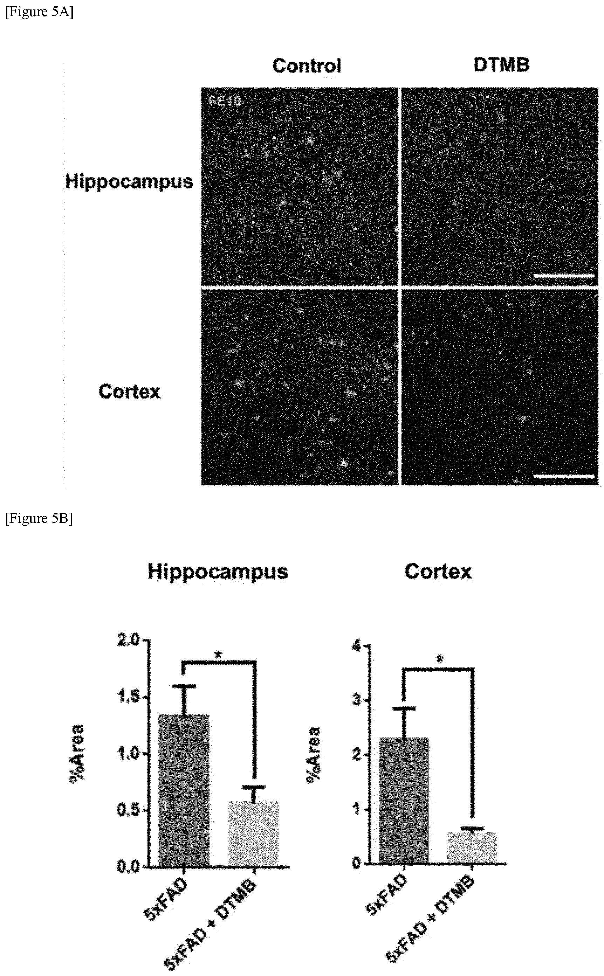

FIG. 5A represents photographs confirming the inhibitory effect of DTMB administration on amyloid aggregate formation in brain tissue (hippocampus and cerebral cortex) in the 5xFAD Alzheimer's dementia animal model using immunohistochemistry, and FIG. 5B shows a graph quantifying the results of FIG. 5A. All data are presented as the mean ± standard error of the mean (SEM) (n = 25 per group). The p-values were indicated as follows based on t-test: **p < 0.01, ***p < 0.001.

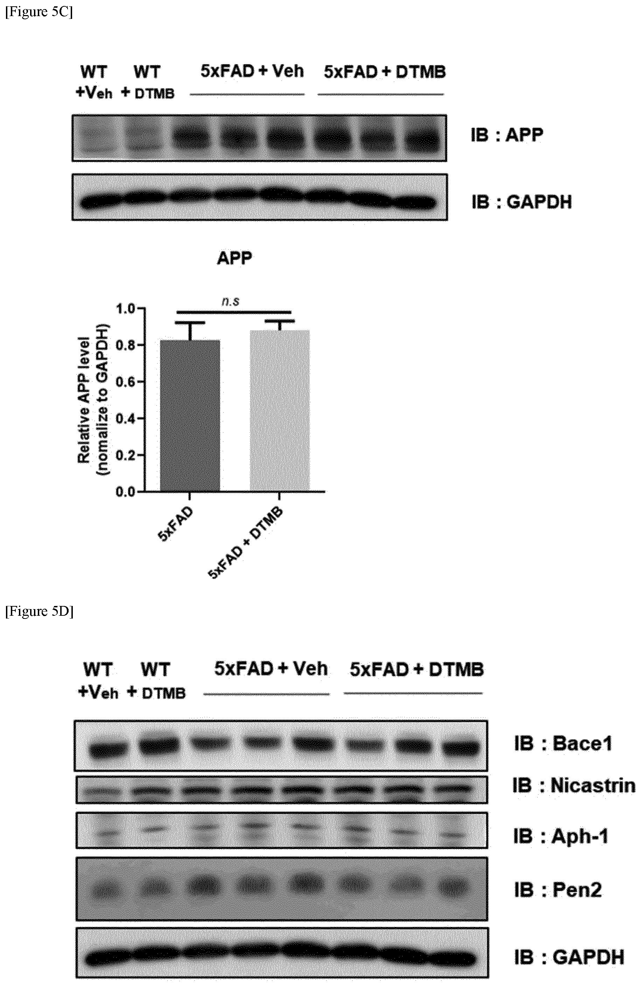

FIG. 5C represents the results of Western blotting confirming the total APP levels in hippocampal tissue following DTMB administration in the 5xFAD Alzheimer's dementia animal model. All data are presented as the mean ± standard error of the mean (SEM) (n = 25 per group). The p-values were indicated as follows based on t-test: **p < 0.01, ***p < 0.001.

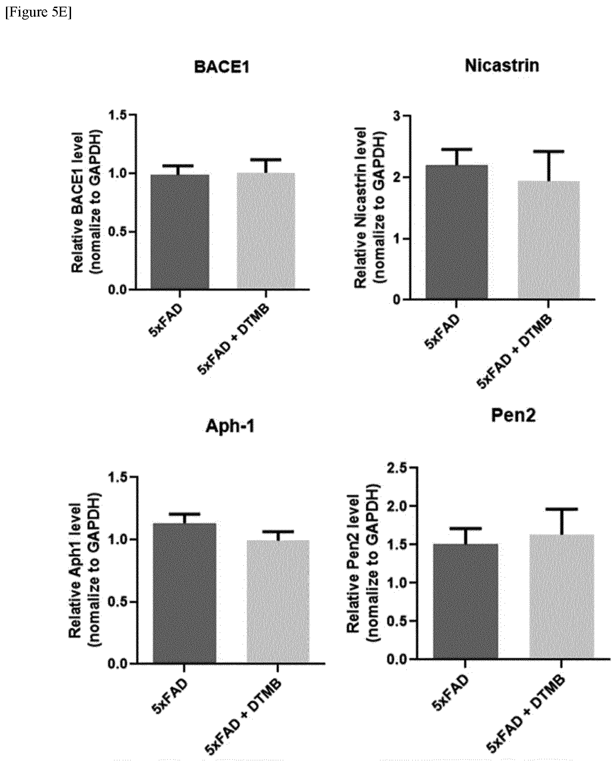

FIG. 5D and FIG. 5E represent the results of Western blotting confirming the protein levels of enzymes related to APP processing (Bace-1 for β-secretase, and Aph1, Nicastrin, and Pen2 for γ-secretase) in hippocampal tissue following DTMB administration in the 5xFAD Alzheimer's dementia animal model. All data are presented as the mean ± standard error of the mean (SEM) (n = 25 per group). The p-values were indicated as follows based on t-test: **p < 0.01, ***p < 0.001.

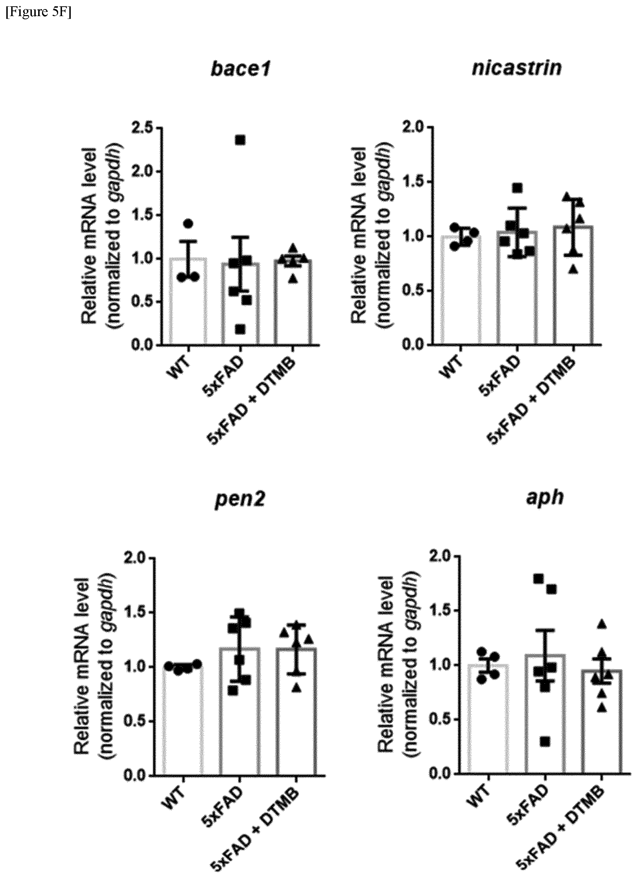

FIG. 5F represents the results of quantitative PCR analysis of gene expression levels of β-secretase (Bace-1) and γ-secretase (Aph1, Nicastrin, and Pen2) in hippocampal tissue following DTMB administration in the 5xFAD Alzheimer's dementia animal model. All data are presented as the mean ± standard error of the mean (SEM). The p-values were indicated as follows based on t-test: **p < 0.01, ***p < 0.001.

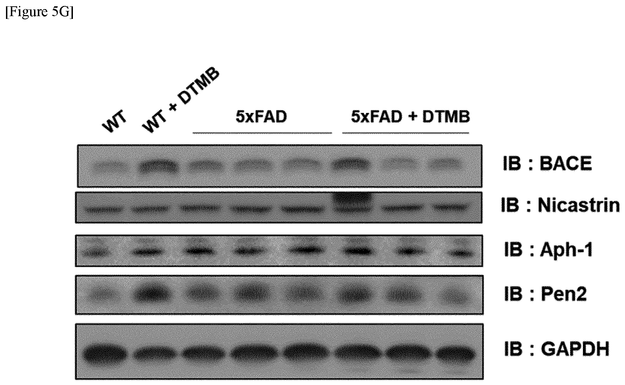

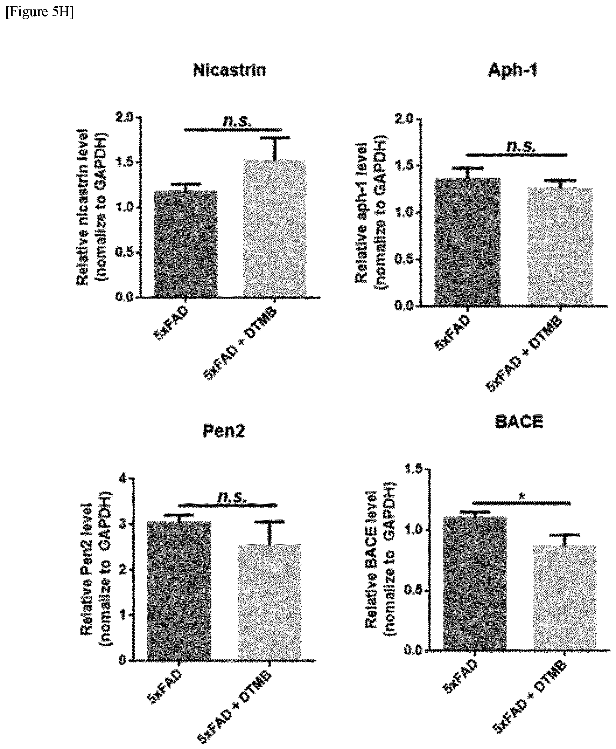

FIG. 5G and FIG. 5H represent the results of Western blotting confirming the protein levels of enzymes related to APP processing (Bace-1 for β-secretase, and Aph1, Nicastrin, and Pen2 for γ-secretase) in lysate samples from cortical tissue following DTMB administration in the 5xFAD Alzheimer's dementia animal model. All data are presented as the mean ± standard error of the mean (SEM). The p-values were indicated as follows based on t-test: **p < 0.01, ***p < 0.001.

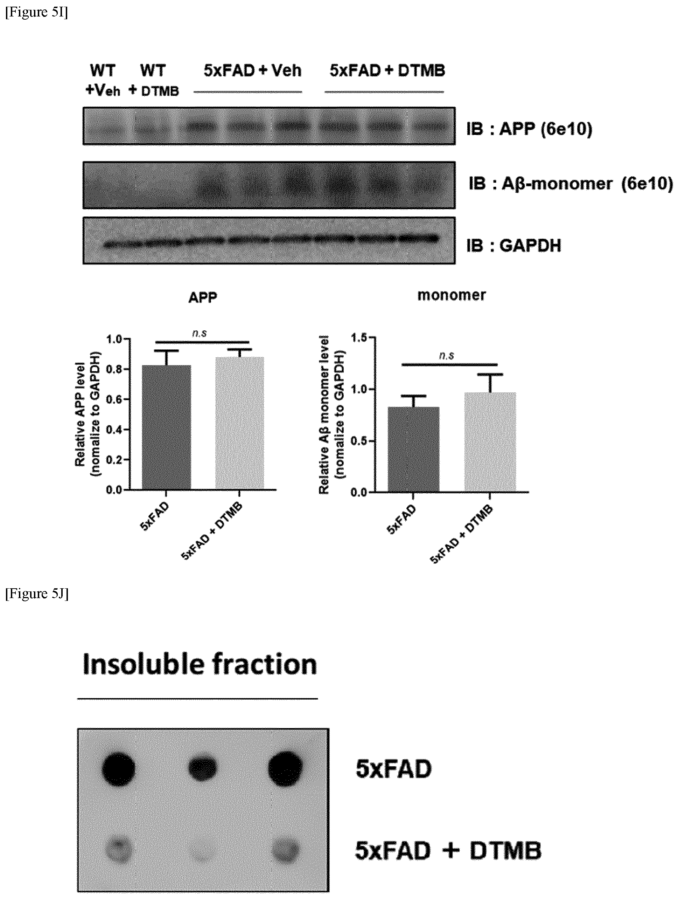

FIG. 5I represents the results of Western blotting confirming the levels of Aβ monomers in brain tissue lysates using the 6e10 antibody following DTMB administration in the 5xFAD Alzheimer's dementia animal model.
FIG. 5J represents dot blot data showing the levels of Aβ plaques in the insoluble fraction of brain tissue lysates following DTMB administration in the 5xFAD Alzheimer's dementia animal model.

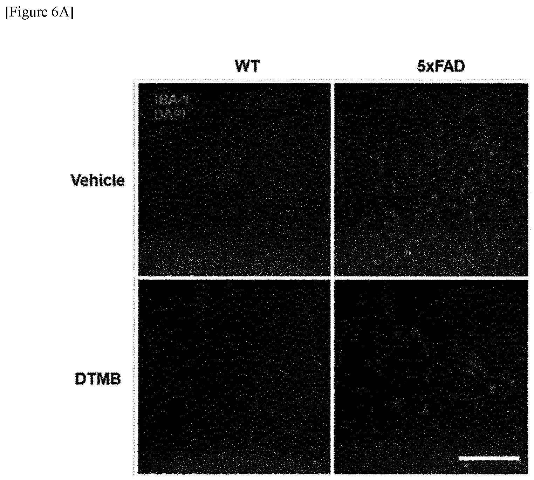

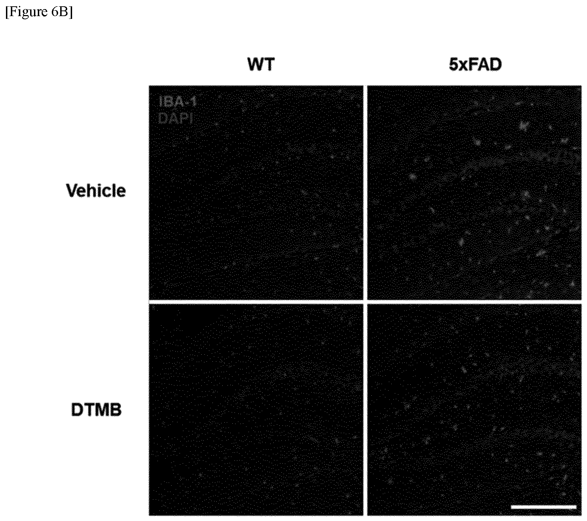

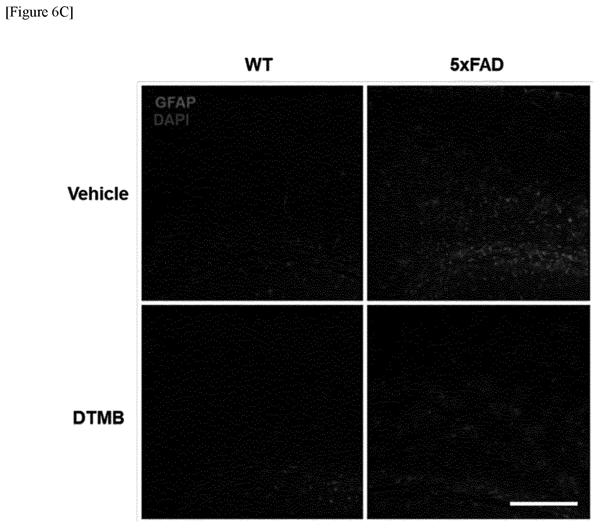

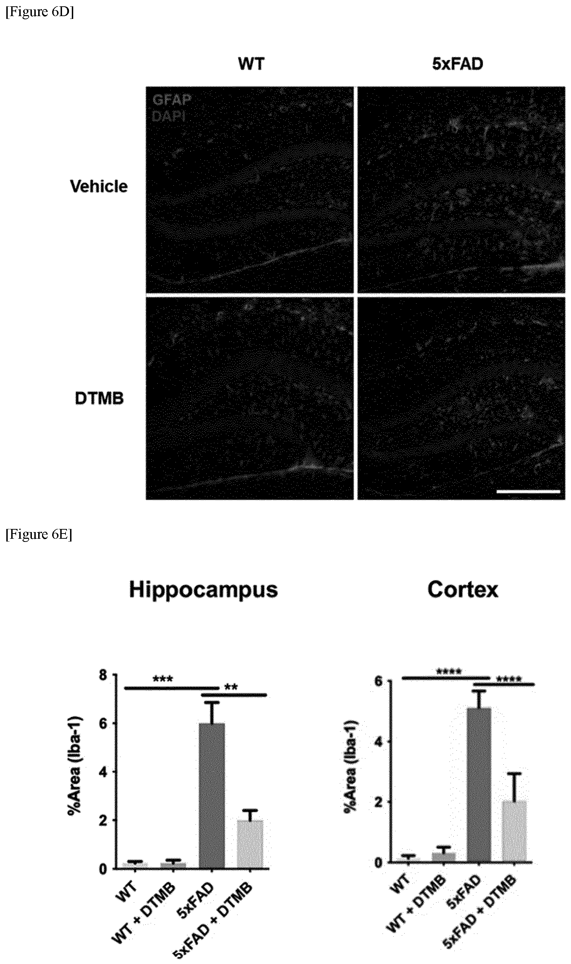

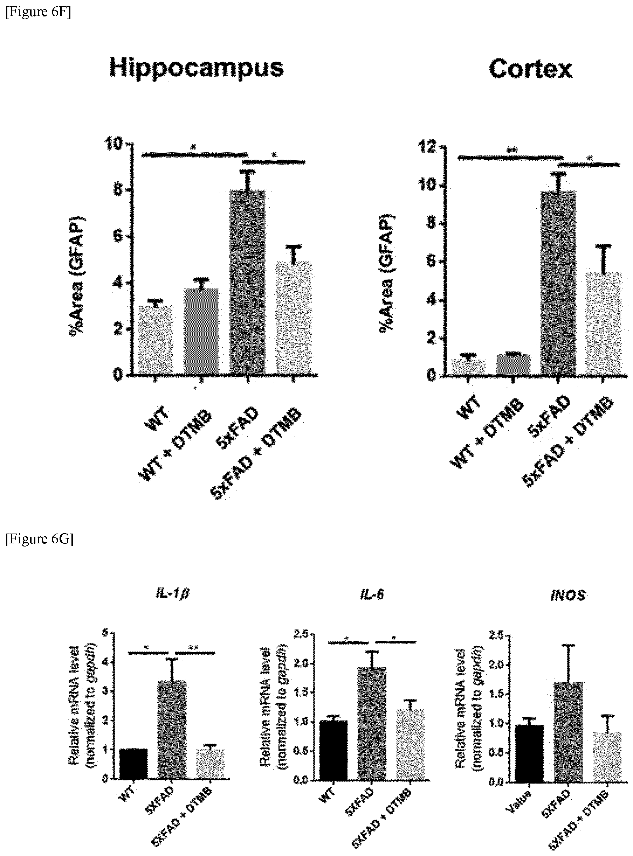

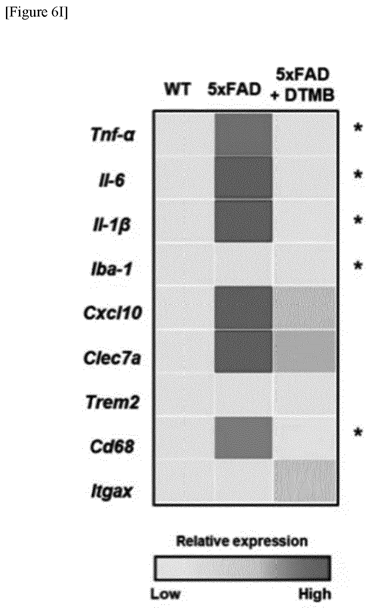

FIGS. 6A to 6F represent the results measuring the reduction in gliosis caused by microglia and astrocytes in brain tissue following DTMB administration in the 5xFAD Alzheimer's dementia animal model, where FIG. 6A and FIG. 6C show the measurement results in the cerebral cortex, FIG. 6B and FIG. 6D show the measurement results in the hippocampus, FIG. 6E represents graphs quantifying the inhibitory effects on microglial hyperactivation in the hippocampus and cerebral cortex, and FIG. 6F represents graphs quantifying the inhibitory effects on astrocyte hyperactivation in the hippocampus and cerebral cortex. All data are presented as the mean ± standard error of the mean (SEM). The p-values were indicated as follows based on one-way ANOVA and t-test: **p < 0.01, ***p < 0.001.
FIG. 6G represents quantitative PCR data for pro-inflammatory cytokines and related enzymes in brain tissue following DTMB administration in the 5xFAD Alzheimer's dementia animal model. All data are presented as the mean ± standard error of the mean (SEM). The p-values were indicated as follows based on one-way ANOVA and t-test: **p < 0.01, ***p < 0.001.

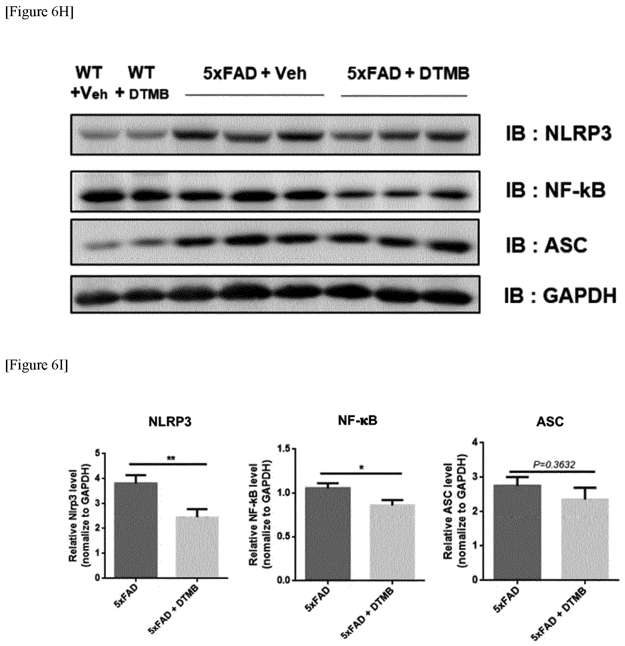

FIG. 6H and FIG. 6I represent the results of Western blotting confirming the levels of inflammasome-related proteins in hippocampal tissue lysates following DTMB administration in the 5xFAD Alzheimer's dementia animal model. All data are presented as the mean ± standard error of the mean (SEM). The p-values were indicated as follows based on one-way ANOVA and t-test: **p < 0.01, ***p < 0.001.
FIG. 7 represents the results showing the expression pattern changes of inflammation-related genes (TNF-α, Il-6, IL-1β, Cxcl10, and clec7a) and microglia activation-related genes (Iba-1, Trem2, and Itgax2) in microglia from brain tissue following DTMB administration in the 5xFAD Alzheimer's dementia animal model. The data represent the mean ± standard error of the mean (SEM) from three independent experiments. The p-values were indicated as follows based on one-way ANOVA: *p < 0.05; **p < 0.01; ***p < 0.001.

### [Best Mode]

As described above, the currently known therapeutics for Alzheimer's dementia have either not demonstrated clear efficacy in treatment or are effective only in alleviating symptoms, leading to a continued demand for the development of therapeutics that can address the fundamental causes. In response, the present inventors, based on previous reports that a pan-agonist capable of activating all three subtypes of PPAR may have therapeutic effects on Alzheimer's dementia, screened novel synthetic small-molecule compounds capable of regulating PPAR activity and experimentally verified that the screened compounds exhibit preventive, amelioration, and treatment effects on degenerative brain diseases, and enhancing effect on learning ability, improving effect on memory, and ameliorating effect on cognitive functions, thereby seeking solutions to the aforementioned problems.

Accordingly, the first aspect of the present invention relates to a pharmaceutical composition for preventing or treating degenerative brain diseases, comprising a compound represented by Chemical Formula 1 below, a stereoisomer thereof or a pharmaceutically acceptable salt thereof as an active ingredient, and a health functional food composition for preventing or ameliorating degenerative brain diseases comprising the same active ingredient.

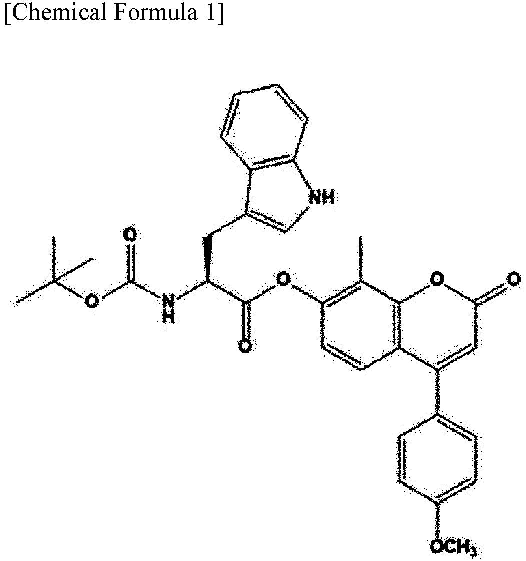

In relation to the first aspect, the present invention also provides a method for preventing, ameliorating or treating degenerative brain diseases, comprising administering a compound represented by Chemical Formula 1 above, a stereoisomer thereof, or a pharmaceutically or sitologically salt thereof to a subject in need thereof.

Additionally, the first aspect of the present invention provides the use of a compound represented by Chemical Formula 1 above, a stereoisomer thereof, or a pharmaceutically or sitologically salt thereof for the manufacture of a medicament for preventing, ameliorating or treating degenerative brain diseases.

The compound represented by Chemical Formula 1 is [4-(4-Methoxyphenyl)-8-methyl-2-oxochromen-7-yl](2S)-3-(1H-indol-3-yl)-2-[(2-methylpropan-2-yl)oxycarbonylamino]propanoate (DTMB) with CAS No: 956044-42-1, which was identified as a novel modulator of PPAR transcription factors capable of activating all three subtypes of PPAR (PPARα, PPARβ/δ, and PPARγ) through analysis using a computer molecular prediction program and GAL4-transactivation assay.

In addition, the stereoisomer may include a racemate, an enantiomer, a diastereomer, a mixture of enantiomers, or a mixture of diastereomers.

The degenerative brain diseases may include one or more selected from the group consisting of Dementia, Autism, Alzheimer's disease, Huntington's disease, Vascular dementia, Stroke, Ischemic stroke, Traumatic brain injury, Amnesia, Parkinson's disease, Pick disease, Creutzfeldt-Jakob disease, Peripheral neuropathy, Amyotrophic lateral sclerosis, multiple sclerosis and mild cognitive impairment, but are not limited thereto.

It has been reported that all three subtypes of PPAR inhibit inflammatory responses through the suppression of NF-κB transcription factor activity and quantitative changes in the NF-κB transcription factor, a key regulator of inflammatory responses. Accordingly, in a specific embodiment of the present invention, to verify whether DTMB, identified as a novel modulator of PPAR transcription factors, exhibits anti-inflammatory effects, RAW264.8 macrophage cell lines were treated with LPS to induce inflammatory responses, followed by treatment with DTMB or known PPAR modulators (WY14643, Rosiglitazone, and GW501516) to evaluate the inhibitory effect on NO production. As shown in FIG. 2b, DTMB inhibited NO production in a concentration-dependent manner, with an IC₅₀ value of 1.23 µM, which was remarkably low. Notably, DTMB inhibited NO production more effectively than WY14643 and Rosiglitazone at the same concentration, confirming its superior anti-inflammatory effects.

Degenerative brain diseases, including Alzheimer's dementia, are characteristically associated with chronic inflammation in brain tissue.

Beta-amyloid (Aβ: amyloid beta) is derived from amyloid precursor protein (APP), which is cleaved by β-secretase and γ-secretase to generate Aβ, and is the main component of amyloid plaques commonly found in the brains of patients with neurological disorders such as Alzheimer's disease. When abnormally accumulated, it becomes a causative factor for various brain diseases. In addition, excessively accumulated beta-amyloid triggers inflammatory responses in the hippocampus, cerebral cortex, and surrounding cells, leading to neuronal damage and potentially disrupting neural networks essential for maintaining normal brain functions.

Accordingly, in another specific embodiment of the present invention, to verify the anti-inflammatory effects of DTMB in neuronal cells, inflammatory responses were induced in BV2 microglial cell lines and primary microglia isolated from mouse by treating them with LPS or Aβ aggregates, followed by treatment with DTMB to measure the levels of cytokines (TNF-α and IL-6). As shown in FIGS. 2C to 2F, DTMB treatment statistically significantly reduced the levels of TNF-α and IL-6 in BV2 microglial cell lines and primary microglia treated with LPS or Aβ aggregates, thereby inhibiting inflammatory responses.

In another specific embodiment of the present invention, to determine the mechanism by which DTMB exerts its anti-inflammatory effects, microglia isolated from mouse were treated with Aβ aggregates to induce an inflammatory response, and then treated with DTMB to determine the levels of NF-κB and NLRP3 which are components of inflammasomes, and ASC (Apoptosis-associated speck like protein containing a caspase recruitment domain), which acts as its adaptor. As shown in FIGS. 2H and 2I, NF-κB and NLRP3 levels, which were elevated by Aβ aggregate treatment, were statistically significantly reduced by DTMB treatment, demonstrating that DTMB inhibits inflammatory responses in microglia.

In another specific embodiment of the present invention, to determine whether DTMB alleviates the pathological symptoms of Alzheimer's dementia, changes in learning ability, memory, and cognitive function following DTMB administration were analyzed in 5xFAD Alzheimer's disease-induced mouse using the Y-maze test and Morris water maze test, as designed in FIG. 3A. As shown in FIG. 3C and FIGS. 4A to 4C, compared to normal mouse, 5xFAD mouse exhibited reduced short-term and long-term memory and decreased cognitive function, but DTMB administration improved short-term and long-term memory and cognitive function to levels equivalent to those of normal mouse.

In another specific embodiment of the present invention, to confirm the inhibitory effect of DTMB administration on Aβ aggregate formation, the amount of Aβ aggregates in the hippocampus and cortex regions of brain tissue from 5xFAD mouse treated with DTMB was measured using immunohistochemistry. As shown in FIG. 5A and FIG. 5B, the increased Aβ aggregates in the hippocampus and cortex regions of brain tissue from 5xFAD mouse not treated with DTMB were significantly reduced by DTMB administration.

The activation of microglia and astrocytes has been reported to be associated with the onset and progression of degenerative degenerative brain diseases, and microglia are immune cells residing in the central nervous system (CNS) and are known to be activated by external stimuli, triggering immune and inflammatory responses. As primary immune cells in the CNS, microglia maintain a slender, elongated shape with thin cell bodies but transform into an activated form with thick, short branches and a swollen cell body in the presence of toxins introduced externally or generated internally, aiming to protect neurons from these toxins.

However, when microglia are stimulated by substances such as LPS, interferon-γ, beta-amyloid, or gangliosides, which are bacterial endotoxins, unlike normal microglia, they exhibit active phagocytosis, proliferate, and express genes such as cytokines including TNF-α, IL-1β, and IL-6, and chemokines, iNOS (inducible nitric oxide synthase), and COX-2 (cyclooxygenase-2), thereby producing inflammatory mediators. The activation of microglia has the aspect of removing damaged cells and protecting neurons from invading bacteria or viruses, but nitric oxide (NO) produced by iNOS, prostaglandins produced by COX-2, and TNF-α exhibit toxicity to neurons, and consequently, excessive activation of microglia exacerbates neuronal damage. Therefore, appropriately suppressing the activation of microglia could be another approach for treating degenerative brain diseases.

In addition, astrocytes play an important role in brain development and in maintaining normal brain function. The astrocytes in the brain have been found to assist neuronal activity by appropriately removing neurotransmitters secreted by neurons or regulating ion concentrations in the brain, and they have also been found to play a crucial role in the differentiation of neural stem cells into neurons.

However, when the brain is injured, astrocytes exhibit increased proliferation and swelling, becoming activated as reactive astrocytes, such as in astrogliosis. These reactive astrocytes have been observed in conditions such as AIDS-related dementia, brain injuries, ischemic brain diseases, and Alzheimer's disease. Therefore, continuous activation of astrocytes ultimately leads to neuronal apoptosis, and appropriately suppressing the activation of astrocytes could also be another approach for treating degenerative brain diseases.

Accordingly, in another specific embodiment of the present invention, to confirm whether DTMB administration inhibits inflammatory responses in brain tissue, the degree of gliosis was analyzed by treating brain tissue from 5xFAD mouse administered with DTMB with antibodies against IBA-1, a microglia-specific protein, and GFAP, an astrocyte-specific protein. As shown in the results of FIGS. 6A to 6F, compared to normal mouse, the cortex (FIGS. 6A, 6C, 6E, and 6F) and hippocampus (FIGS. 6B, 6D, 6E, and 6F) regions of 5xFAD mouse not administered with DTMB exhibited a rapid increase in gliosis due to excessive activation of microglia and astrocytes, but in the cortex (FIGS. 6A, 6C, 6E, and 6F) and hippocampus (FIGS. 6B, 6D, 6E, and 6F) regions of 5xFAD mouse administered with DTMB, gliosis was significantly reduced.

In another specific embodiment of the present invention, to confirm whether the reduction in gliosis following DTMB administration is attributable to changes in microglial function, microglia were isolated from the brain tissue of 5xFAD mouse administered with DTMB, and the expression levels of cytokine genes (TNF-α, Il-6, and IL-1β) related to inflammatory responses, chemokine genes (Cxcl10 and clec7a), and genes associated with microglial activation (Iba-1, Trem2, and Itgax2) were analyzed. As shown in FIG. 10, the expression levels of cytokine genes (TNF-α, Il-6, and IL-1β), chemokine genes (Cxcl10 and clec7a), and genes associated with microglial activation (Iba-1, Trem2, and Itgax2) were all increased in microglia from 5xFAD mouse compared to normal mouse, but in microglia from 5xFAD mouse administered with DTMB, the expression levels of these genes were decreased. This confirms that DTMB can suppress excessive activation of microglia.

Accordingly, the compound represented by Chemical Formula 1 of the present invention, the stereoisomer thereof, or a pharmaceutically or sitologically salt thereof may exhibit one or more activities selected from the following i) to iii):
i) inhibiting inflammatory responses by activating PPAR transcription factors in microglia;
ii) inhibiting the formation of Aβ aggregates in brain tissue; and
iii) inhibiting gliosis caused by excessive activity of microglia and astrocytes.

The effects of DTMB as described above may have a positive impact on ameliorating degenerative brain diseases, impaired learning ability, memory loss, or cognitive decline caused by Aβ aggregate deposition and/or neuroinflammatory responses, and accordingly, the compound represented by Chemical Formula 1 of the present invention, stereoisomer thereof, or a pharmaceutically or sitologically salt thereof can be utilized not only as a pharmaceutical or health functional food for preventing, ameliorating or treating degenerative brain diseases but also as a pharmaceutical or health functional food for enhancing learning ability, improving memory, or ameliorating cognitive decline.

Accordingly, the second aspect of the present invention relates to a composition for the enhancing learning ability, improving memory, or ameliorating cognitive function, comprising a compound represented by Chemical Formula 1, a stereoisomer thereof, or a pharmaceutically or sitologically salt thereof as an active ingredient.

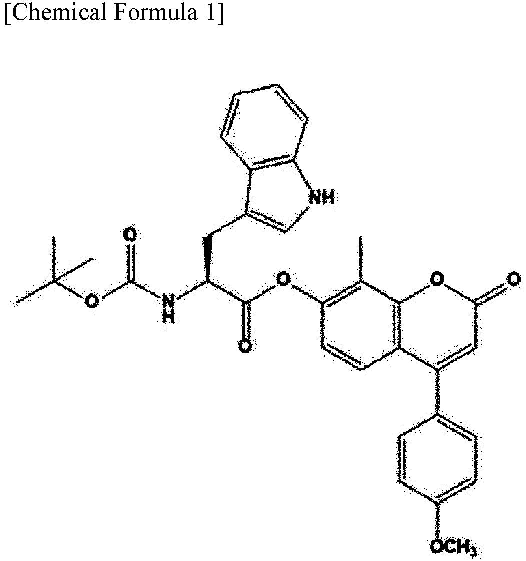

In relation to the second aspect, the present invention also provides a method for enhancing learning ability, improving memory or ameliorating cognitive decline, comprising administering a compound represented by Chemical Formula 1 above, a stereoisomer thereof or a pharmaceutically or sitologically salt thereof to a subject in need thereof.

Additionally, the second aspect of the present invention provides the use of a compound represented by Chemical Formula 1 above, stereoisomers thereof or pharmaceutically or sitologically salts thereof for the manufacture of a medicament for enhancing learning ability, improving memory or ameliorating cognitive decline.

The composition and effects of the compound represented by Chemical Formula 1, stereoisomers thereof, or a pharmaceutically or sitologically salt thereof, which is included as an active ingredient in the composition according to the second aspect of the present invention, are identical to those described for the pharmaceutical composition and health functional food composition according to the first aspect, and therefore, a detailed description thereof will be omitted.

The composition for enhancing learning ability, improving memory, or ameliorating cognitive function according to the present invention may be a pharmaceutical composition or a health functional food composition.

In the present invention, the terms "improving memory" and "improving cognitive function" refer to the effects of reducing memory loss, memory impairment, or cognitive decline that occurs when the brain atrophies and destroys brain nerve cells due to physical fatigue, lack of sleep, excessive alcohol consumption, dementia, etc. and maintaining cognitive function by controlling harmful substances that damage brain cells, or ameliorating cognitive decline by controlling neurotransmitters in the brain. Memory refers to the ability to accept information, store it in the brain, and retrieve it when needed, and cognitive ability refers to the capacity to distinguish and recognize objects.

In the present invention, the term "prevent" refers to any act that inhibits or delays the onset of a disease or pathology. In the context of the present invention, it means delaying the onset of a degenerative brain disease or inhibiting its development. Alternatively, it means delaying or inhibiting the onset of symptoms of learning difficulties, memory loss, or cognitive decline.

In the present invention, the term "ameliorate" means any act that alleviates or beneficially alters a disease or pathological condition, and in the context of the present invention, alleviates the symptoms of a degenerative brain disease. Alternatively, it means improving learning, memory, and cognitive function.

In the present invention, the term "treat" means any act that delays, halts, or reverses the progression of a disease or pathology, and in the context of the present invention, means reducing, alleviating, eliminating, or reversing the symptoms of a degenerative brain disease. Alternatively, it means reducing, alleviating, eliminating, or reversing the symptoms of learning difficulties, memory loss, or cognitive decline.

In the present invention, the term "pharmaceutically or sitologically salt" means any organic or inorganic prodrug salt of a compound of Formula 1 or a stereoisomer thereof at a concentration that is relatively non-toxic and harmless to a patient, such that adverse effects attributable to the salt do not detract from the beneficial effects of the compound of Formula 1 or a stereoisomer thereof. These salts can be made from inorganic and organic acids, with inorganic acids including hydrochloric acid, bromic acid, nitric acid, sulfuric acid, perchloric acid, phosphoric acid, and organic acids including citric acid, acetic acid, lactic acid, maleic acid, fumaric acid, gluconic acid, and methanesulfonic acid, glycolic acid, succinic acid, tartaric acid, galacturonic acid, embonic acid, glutamic acid, aspartic acid, oxalic acid, (D) or (L) malic acid, maleic acid, methanesulfonic acid, ethanesulfonic acid, 4-toluenesulfonic acid, salicylic acid, citric acid, benzoic acid, or malonic acid. The salts also include alkali metal salts (sodium salts, potassium salts, etc.) and alkaline earth metal salts (calcium salts, magnesium salts, etc.). For example, birth salts include acetate, aspartate, benzate, besylate, bicarbonate/carbonate, bisulfate/sulfate, borate, campsylate, citrate, edicylate, esylate, formate, and fumarate, Gluceptate, Gluconate, Glucuronate, Hexafluorophosphate, Hibenzate, Hydrochloride/Chloride, Hydrobromide/Bromide, Hydroiodide/Iodide, Isethionate, Lactate, Malate, Malyate, malonate, mesylate, methyl sulfate, naphthylate, 2-naphthylate, nicotinate, nitrate, orotate, oxalate, palmitate, pamoate, phosphate/hydrogen phosphate/dihydrogen phosphate, saccharate, stearate, succinates, tartrates, tosylates, trifluoroacetates, aluminum, alginine, benzathine, calcium, choline, diethylamine, diolamine, glycine, lysine, magnesium, meglumine, olamine, potassium, sodium, tromethamine, zinc salts, and the like.

The pharmaceutical compositions of the present invention may further comprise a pharmaceutically acceptable carrier. The pharmaceutical composition comprising a pharmaceutically acceptable carrier may be in various oral or parenteral formulations. When formulated, they may be prepared using diluents or excipients such as fillers, bulking agents, binders, wetting agents, disintegrating agents, surfactants, and the like in common use. Solid dosage forms for oral administration may include tablets, pills, acid tablets, granules, capsules, troches, and the like, which may be formulated by mixing one or more compounds of the invention with at least one excipient, for example, starch, calcium carbonate, sucrose or lactose, or gelatin. In addition to simple excipients, lubricants such as magnesium stearate talc may also be used. Liquid preparations for oral administration may be suspensions, solutions, emulsions, or syrups, and may contain a variety of excipients, such as wetting agents, sweeteners, flavorings, and preservatives, in addition to the commonly used simple diluents of water and liquid paraffin.

Formulations for parenteral administration may include sterile aqueous solutions, non-aqueous solutions, suspensions, emulsions, lyophilizates, suppositories, and the like. Non-aqueous solvents and suspensions may include propylene glycol, polyethylene glycol, vegetable oils such as olive oil, and injectable esters such as ethylolate. As a base for suppositories, Witepsol, Macrogol, Tween 61, cacao butter, laurinum, glycerol, gelatin, etc. can be used.

The pharmaceutical compositions of the present invention may provide a desirable prevention, amelioration or treatment effect of a degenerative brain diseases, an enhancement effect of learning, an improvement effect of memory or an amelioration effect of cognitive decline when comprising an effective amount of a compound represented by Chemical Formula 1, a stereoisomer thereof or a pharmaceutically or sitologically salt thereof. As used herein, the term "effective amount" refers to an amount that produces a response greater than or equal to a negative control, preferably an amount sufficient to prevent, ameliorate, or treat a degenerative brain diseases, or an amount sufficient to improve learning ability, memory or cognitive decline. The pharmaceutical composition of the present invention may include a compound represented by Chemical Formula 1, a stereoisomers thereof, or pharmaceutically or sitologically acceptable salts thereof in an amount ranging from 0.01% to 99.9%, with the remainder comprising pharmaceutically acceptable carriers. The effective amount of the compound represented by Chemical Formula 1, a stereoisomers thereof, or pharmaceutically or sitologically salts included in the pharmaceutical composition may vary depending on the form in which the composition is commercialized.

The total effective amount of the pharmaceutical composition of the present invention may be administered to a patient as a single dose or via a fractionated treatment protocol involving multiple doses administered over an extended period. The pharmaceutical composition of the present invention may vary in the content of active ingredients depending on the severity of the disease. The dosage of the compound represented by Chemical Formula 1, a stereoisomer thereof, or a pharmaceutically or sitologically salt thereof is determined based on an effective dosage for the patient, taking into consideration various factors such as the administration route of the pharmaceutical composition, frequency of treatment, and the patient's age, weight, health condition, gender, severity of the disease, diet, and excretion rate, and given these considerations, a person skilled in the art would be able to determine an appropriate effective dosage of the compound represented by Chemical Formula 1, a stereoisomer thereof, or a pharmaceutically or sitologically salt thereof for specific uses, such as the prevention, amelioration, or treatment of degenerative brain diseases, or for the enhancement of learning ability, improvement of memory, or amelioration of cognitive decline. The pharmaceutical composition according to the present invention is not particularly limited in its formulation, administration route, or method of administration, as long as it exhibits the effects of the present invention.

The pharmaceutical composition of the present invention may be administered via any general route that allows it to reach the target tissue or cells within a subject or sample. The administration may include systemic or local administration and may encompass intraperitoneal, intravenous, intramuscular, subcutaneous, intradermal, oral, intranasal, pulmonary, and rectal administration, but is not limited thereto. The dosage of the pharmaceutical composition may vary depending on the patient's age, body weight, gender, administration method, health condition, and the severity of the disease.

The term "patient" refers to any single individual requiring treatment, including humans, cows, dogs, guinea pigs, rabbits, chickens, insects, and the like. Additionally, it includes any subject participating in clinical research trials without exhibiting clinical signs of disease, subjects involved in epidemiological studies, or those used as control groups.

In the present invention, the term "health functional food" encompasses the meanings of both "functional food" and "health food."

In the present invention, the term "Functional food" is synonymous with "Food for special health use (FoSHU)" and refers to food that is processed to efficiently exhibit biological regulatory functions in addition to providing nutrition, with high medical and therapeutic efficacy.

In the present invention, the term "Health food" refers to food that has active health maintenance or promotion effects compared to general food, and "Health supplement food" refers to food intended for health supplementation purposes. In some cases, the terms functional food, health food, and health supplement food are used interchangeably. These foods may be manufactured in various forms such as tablets, capsules, powders, granules, liquids, or pills to achieve beneficial effects for preventing or ameliorating degenerative brain diseases, or for enhancing learning ability, improving memory, or ameliorating cognitive function.

As a specific example of such functional foods, processed foods can be manufactured by utilizing the compound represented by Chemical Formula 1 of the present invention, a stereoisomer thereof, or its sitologically salt, enhancing the characteristics of agricultural, livestock, or marine products while improving their storability.

The health functional food composition of the present invention can also be manufactured in the form of nutritional supplements, food additives, or feed and is intended for consumption by humans or animals, including livestock.

The food compositions of the above type can be manufactured in various forms using conventional methods known in the art. General foods, though not limited thereto, include beverages (including alcoholic beverages), fruits and their processed products (e.g., canned fruits, jarred fruits, jams, marmalades, etc.), fish, meat and their processed products (e.g., ham, sausages, corned beef, etc.), bread and noodles (e.g., udon, soba, ramen, spaghetti, macaroni, etc.), fruit juices, various drinks, cookies, taffy, dairy products (e.g., butter, cheese, etc.), edible vegetable oils, margarine, vegetable proteins, retort foods, frozen foods, and various seasonings (e.g., soybean paste, soy sauce, sauces, etc.), and these can be manufactured by adding a compound represented by Chemical Formula 1, a stereoisomer thereof, or its food-acceptable salt of the present invention.

Additionally, nutritional supplements, though not limited thereto, can be manufactured by adding a compound represented by Chemical Formula 1, a stereoisomer thereof, or its food-acceptable salt of the present invention to capsules, tablets, pills, etc.

Furthermore, although not limited to dietary supplements, for example, the compounds represented by Chemical Formula 1 of the present invention, a stereoisomer thereof, or sitologically salts thereof can be formulated in the form of teas, juices, and beverages and liquefied, granulated, encapsulated, and powdered for consumption (health beverages). Additionally, a compound represented by Chemical Formula 1 of the present invention, a stereoisomer thereof, or its sitologically salt can be prepared in the form of a powder or concentrate for use as a food additive. Additionally, a compound represented by Chemical Formula 1 of the present invention, a stereoisomer thereof, or its sitologically salt can be formulated into a composition by mixing it with known active ingredients that are reported to be effective for preventing or ameliorating degenerative brain diseases, or for enhancing learning ability, improving memory, or ameliorating cognitive function.

When the food composition of the present invention is used as a health beverage composition, the health beverage composition may include additional components such as various flavoring agents or natural carbohydrates, similar to conventional beverages. The aforementioned natural carbohydrates may include monosaccharides such as glucose and fructose; disaccharides such as maltose and sucrose; polysaccharides such as dextrin and cyclodextrin; and sugar alcohols such as xylitol, sorbitol, and erythritol. Sweeteners may include natural sweeteners such as thaumatin and stevia extracts, and synthetic sweeteners such as saccharin and aspartame, etc. The proportion of the natural carbohydrates is generally about 0.01 to 0.04 g per 100 mL of the composition, preferably about 0.02 to 0.03 g.

A compound represented by Chemical Formula 1 of the present invention, a stereoisomer thereof, or its sitologically salt can be included as an active ingredient in a health functional food composition for preventing or ameliorating degenerative brain diseases or for enhancing learning ability, improving memory, or ameliorating cognitive function, and the amount of the compound is preferably an effective amount to achieve the above prevention or amelioration effects, for example, 0.01 to 100% by weight based on the total weight of the composition, but is not particularly limited thereto. The health functional food composition of the present invention can be prepared in the form of a composition by mixing a compound represented by Chemical Formula 1, a stereoisomer thereof, or its sitologically salt with known active ingredients that are reported to be effective for preventing or ameliorating degenerative brain diseases, or for enhancing learning ability, improving memory, or ameliorating cognitive function.

In addition, the health functional food of the present invention may contain various nutrients, vitamins, electrolytes, flavoring agents, colorants, pectic acid, salts of pectic acid, alginic acid, salts of alginic acid, organic acids, protective colloidal thickeners, pH adjusters, stabilizers, preservatives, glycerin, alcohol, or carbonating agents, etc. Furthermore, the health food of the present invention may contain fruit pulp for the production of natural fruit juice, fruit juice beverages, or vegetable beverages. These components may be used independently or in combination. Although the proportion of such additives is not critically important, it is generally selected within a range of 0.01 to 0.1 parts by weight per 100 parts by weight of the composition of the present invention.

In the method of the present invention, the term "subject" includes any animal (e.g., humans, horses, pigs, rabbits, dogs, sheep, goats, non-human primates, cows, cats, guinea pigs, or rodents) but is not limited thereto. This term does not specify a particular age or gender. Accordingly, it is intended to include female/female or male/male, adult/adult and neonatal subjects, and fetuses. A patient refers to a subject affected by a disease or disorder. The term "patient" encompasses both human and veterinary subjects.

In the methods of the present invention, the effects of the compounds represented by Chemical Formula 1, a stereoisomer thereof, or sitologically salts thereof, and the description of the composition, including the route of administration, number of doses, dosage amounts, and the like, are the same as described above and are therefore omitted.

Hereinafter, the invention will be described in more detail by way of example. However, the invention is subject to various modifications and may take many forms, and the specific embodiments and descriptions described below are intended to illustrate the invention and are not intended to limit the invention to any particular disclosed form. The scope of the invention is to be understood to include all modifications, equivalents, or substitutes that fall within the scope of the idea and skill of the present invention.

### [Mode for Invention]

### [Example 1]

### Discovery and Efficacy Analysis of DTMB as a Novel PPAR Transcription Factor Active agent

To develop a novel PPAR active agent, a computer molecular binding prediction program analysis and a GAL4-transactivation assay were simultaneously performed as shown in FIG. 1A to discover DTMB compounds.

### 1-1. Preparation of DTMB compounds

At this time, DTMB was synthesized by Daejung Chemicals & Metals Co., Ltd. (Korea) and used, and was dissolved in dimethyl sulfoxide (Sigma Aldrich, St. Louis, MO, USA) for cell-based experiments.

### 1-2. GAL4-transactivation assay

A GAL4-transactivation assay experiment was performed to determine whether the drug was bound to the PPAR ligand binding site. HEK293A cells were transfected with GAL4-reporter plasmid and pGL4.74 (Promega, Madison, WI, USA) as an internal standard via electroporation using the Neon Transfection System (Invitrogen, Carlsbad, CA, USA). All vectors were kindly provided by Professor Kanno Yuichiro, Toho University, Japan. After overnight culture, cells were treated with DTMB and each positive control compound for 24 h. Cells were then harvested, resuspended in luciferase lysis buffer (Promega), and incubated on ice for 10 min. Cell debris was removed by centrifugation at 20,000 × g, 4 °C for 10 min, and the supernatant was used for the luciferase assay. Luciferase activity was measured using the Dual Luciferase Reporter Assay System (Promega) according to the manufacturer's instructions. In the measurement experiment, firefly luciferase activity was normalized to Renilla luciferase activity. To investigate the concentration-dependent effect of DTMB drug, EC50 values were measured by treating various concentrations of DTMB.

As a result, as confirmed in FIG. 1B, when compared with agents that activate each subtype of PPAR previously reported, DTMB compound showed an increase in the degree of binding to PPAR in a concentration-dependent manner, and PPARα was activated at a lower concentration than WY14643, and PPARβ/δ and PPARγ were activated at a slightly higher concentration than GW501516 and rosiglitazone, respectively.

### 1-3. CNBr-Drug and Protein Conjugation Assay

To screen PPAR active agent, Autodock Vina program and Phymol program were used to predict whether DTMB binds to PPAR transcription factors. The three-dimensional structures of PPAR were provided by RCSB Protein Data Bank and used for the degree of binding prediction (PPARα: 4BCR, 3VI8, 5HYK; PPARδ: 5U3Q, 5U46; PPARγ: 3U9Q, 5YCP, 5JI0). The energies of DTMB or other PPAR agonists (WY14643, GW501516, and rosiglitazone) were minimized using open babel of PyRx software for further docking analysis. Molecular docking and predicted binding energy calculations were performed using AutoDock Vina in PyRx software to find the docking positions of ligands for PPAR. The grid map for docking covered the entire structure of the macromolecule. The docking results were visualized using the PyMol visualization system. The hydrogen bonds were predicted by the PyMol software.

Similar to the luciferase analysis, the molecular docking analysis results predicted that the binding energy between DTMB and the PPARα ligand binding domain (LBD) was higher than that of WY14643, as confirmed in FIG. 1C and Table 1, and DTMB was predicted to hydrogen bond with Ser280 of PPARα LBD, Arg248 and Ala306 of PPARδ, and Ser289 of PPARγ.

**[Table 1]**

| Predicted binding energy of PPARβ/δ/γ and DTMB | | |
|---|---|---|
| Target LBD model of PPARα | Predicted binding energy with WY14643 (ΔG) (kJ mol⁻¹) | Predicted binding energy with DTMB (ΔG) (kJ mol⁻¹) |
| 3vi8 | -7.8 | -8.3 |
| 5hyk | -6.9 | -8.4 |
| 4bcr_A | -7.6 | -8.3 |
| 4bcr_B | -7.6 | -8.6 |

| Target LBD model of PPARδ | Predicted binding energy with GW501516(ΔG) (kJ mol⁻¹) | Predicted binding energy with DTMB (ΔG) (kJ mol⁻¹) |
|---|---|---|
| 5u3q_A | -11.4 | -9.4 |
| 5u46_A | -10.9 | -7.7 |
| 5u46_B | -10.7 | -9.3 |

| Target LBD model of PPARγ | Predicted binding energy with rosiglitazone(ΔG) (kJ mol⁻¹) | Predicted binding energy with DTMB (ΔG) (kJ mol⁻¹) |
|---|---|---|
| 3u9q | -10.7 | -7.7 |
| 5ycp | -9.9 | -8.2 |
| 5ji0 | -10.7 | -8.0 |

Since the computer prediction program predicted that DTMB binds to PPAR transcription factors, CNBr-drug and protein binding experiments were conducted to confirm whether DTMB actually binds to PPAR. To determine the actual degree of drug-protein binding, the reaction of the protein of the PPAR-α, β/δ, γ ligand-binding portion with the DTMB drug-bound CNBr beads was confirmed through Western blotting.

Specifically, CNBr Sepharose 4B beads were prepared and activated according to the instructions. 100 mg of CNBr-activated Sepharose 4B beads were incubated with drugs or DMSO at 4 °C for 18 h. The drug-bound beads reacted with HIS-tagged recombinant PPAR α, β/δ, and γ proteins. After 24 h of incubation, Western blotting experiments were performed to confirm whether the recombinant proteins bound to the drug-bound beads.

As a result, as shown in FIG. 1D, it was found that the DTMB drug binds well to the protein of the PPAR ligand binding portion, and it was confirmed that DTMB binds to all subtypes of PPAR. Through these results, DTMB was suggested as a novel regulator of PPAR transcription factors.

### [Example 2]

### Confirmation of the anti-inflammatory effect of DTMB

### 2-1. Cell viability assay (MTT assay)

Cell viability was measured by a chromogenic assay that biologically reduces 3-(4,5-dimethyl-thiazol-2-yl)-2,5-diphenyltetrazolium bromide (MTT) to formazan in living cells. Briefly, cells were cultured in 96-well plates in a final volume of 100 µL. After 24 h, the culture medium was replaced with fresh medium containing DTMB compound, and the cells were cultured for another 48 h. Next, MTT (dissolved in PBS at 5 mg/mL) was added to the culture medium. The cells were cultured in the dark at 37°C for 1 h, and then the supernatant was removed from the wells. The formazan crystals were dissolved by adding total MTT solvent (4 mM HCl and 0.1% Nonidet P-40, both dissolved in isopropanol), and the absorbance was measured at 570 nm using a microplate reader, Infinite 200 Pro NanoQuant microplate reader (TECAN, Switzerland).

As a result, as confirmed in FIG. 2A, there was no cytotoxicity at higher concentrations when DTMB was treated on immune cells such as RAW 264.7, BV2, and HMO6 cell lines.

### 2-2. Confirmation of NO production inhibition effect of DTMB

Previous studies have reported that all three subtypes of PPAR inhibit inflammatory responses through inhibition of the activity and quantitative changes of the NF-κB transcription factor, a major regulator of inflammatory responses. Accordingly, to determine whether DTMB treatment exhibits an anti-inflammatory effect, RAW264.8 macrophage cells were treated with DTMB and previously known PPAR regulators WY14643 (PPAR-α regulator), rosiglitazone (PPAR-γ regulator), and GW501516 (PPAR β/δ), respectively, and the amount of NO, a product of the inflammatory response, was measured using the Griess reagent.

First, RAW 264.7 macrophages were cultured in a humidified atmosphere of 37°C in a 5% CO₂ incubator with DMEM containing 10% FBS and 1% penicillin/streptomycin. After inducing an inflammatory response in RAW 264.7 macrophages by LPS treatment, the Griess assay (Promega) was performed according to the manufacturer's instructions to measure the concentration of nitrite. Known PPAR selective agonists such as WY14643 (PPARα), GW501516 (PPAR-β/δ), and rosiglitazone (PPARγ) were used as positive controls.

As a result, as shown in FIG. 2B, DTMB inhibited NO production in a concentration-dependent manner, and the IC50 value was very low at 1.23 µM. In addition, it was confirmed to inhibit NO production more effectively than WY14643, a PPARα modulator, and rosiglitazone, a PPARγ modulator, at the same concentration.

### 2-3. Confirmation of cytokine reduction effect in microglia by DTMB treatment

To confirm a more clear anti-inflammatory effect, the microglia cell line (BV2) and microglia isolated from mice were treated with lipopolysaccharide (LPS) and Aβ aggregates to induce an inflammatory response, and then DTMB was treated to measure the decrease in cytokine levels in microglia by DTMB treatment using ELISA.

The BV2 mouse microglia cell line was provided by Professor Seok Kyung-ho of Kyungpook National University. BV2 cells were cultured in a 5% CO₂ incubator with a humidified atmosphere at 37°C with Dulbecco's Modified Eagle Medium (DMEM) (Hyclone, Logan, UT, USA) containing 5% fetal bovine serum (FBS, Hyclone) and 50 µg/mL gentamicin (Lonza, Switzerland).

Primary microglial culture was performed as described in the literature [Lian H, Roy E, Zheng H. Protocol for primary microglial culture preparation. Bio-protocol. 2016;6(21)]. Postnatal day 3 mice were used for primary microglial cell preparation. Newborn mouse pups were anesthetized on ice and then sacrificed. The isolated cortex and hippocampus were collected after removing the meninges. The brain tissues were dissociated by incubation with trypsin (2.5%) in HBSS (Gibco, Gaithersburg, MD, USA) for 15 min. The cell homogenate was then cultured in M-CSF (20 ng/mL, R&D Systems) supplemented with DMEM-F12 medium containing 10% fetal bovine serum, 10% horse serum, 1% GlutaMAX (Gibco, MD, USA), and 1% penicillin/streptomycin (Welgene, Korea). After 10 days, microglia were purified by shaking at 260 rpm for 2 h.

Cytokine levels (TNF-α, IL-1β, and IL-6) were measured in the media of BV2 microglia and primary microglia treated with LPS (1 µg/mL) and human Aβ1-42 (5 µg/mL). Human Aβ1-42 (AnaSpec, Fremont, CA, USA) was pre-aggregated in the culture medium for 24 h before cell treatment. BV2 cells and primary microglia were seeded in 12-well plates (SPL) at a density of 1 × 10⁵ cells/well in 1 mL of complete medium. After 12 h, cells were treated with LPS or human Aβ1-42 and further cultured with DTMB for 24 h. ELISA experiments were performed according to the manufacturer's instructions using the mouse TNF-α ELISA kit (Invitrogen, BMS607-3TEN), mouse IL-1β ELISA kit (Invitrogen, BMS6002), and mouse IL-6 ELISA kit (Invitrogen, BMS603-2).

As a result, as shown in the results of FIGS. 2C to 2F, it was confirmed that the treatment with DTMB statistically significantly inhibited the inflammatory response.

In addition, the gene expression levels of *Il-6, Il-1#,* and *iNOS,* which are NF-κB target genes related to chronic inflammation, and Arg-1, an anti-inflammatory marker, were confirmed by quantitative PCR (qPCR) analysis in primary microglia.

Total RNA was isolated using TRI reagent (Molecular Research Center, Cincinnati, OH, USA). RNA was reverse transcribed using the ImProm-II Reverse Transcription System (Promega) according to the manufacturer's instructions. For detection and quantification, the StepOnePlus Real-Time PCR System (Applied Biosystems, Foster City, CA, USA) was used with FastStart Universal SYBR Green Master (Roche, Basel, Switzerland). Real-time qPCR data were analyzed using the comparative CT method.

As shown in the results in FIG. 2G, the expression of Il-6, Il-1β, and iNOS, which are NF-κB target genes associated with chronic inflammation, was decreased in primary microglia. In contrast, the anti-inflammatory marker (Arg-1) was induced by DTMB treatment.

### 2-4. Confirmation of the anti-inflammatory activity mechanism of DTMB

To confirm the mechanism by which DTMB exerts its anti-inflammatory effect, microglial cells isolated from mice were treated with Aβ aggregates or LPS to induce an inflammatory response, and then DTMB was treated to determine the levels of NF-κB, NLRP-3, and ASC proteins by western blotting.

The cells were lysed with lysis buffer containing 50 mM Tris (pH 7.4), 140 mM NaCl, 5 mM EDTA, and protease inhibitor tablets, and then sonicated. The protein concentration of the lysate was determined using Bradford reagent (AMERSCO, MA, USA). Proteins were separated by sodium dodecyl sulfate (SDS) polyacrylamide gel electrophoresis, transferred to nitrocellulose membranes (Pall Corporation, New York, NY, USA), and incubated with blocking buffer (5% nonfat dry milk and 0.1% Tween 20 in TBS) for 30 min. Western blot analysis was performed using primary antibodies against NF-κB, NLRP3, and ASC (Cell Signaling Technology, Danvers, MA, USA). Secondary antibodies from rabbit (Promega), rat, and goat (Bethyl Laboratories) were used, and detection was performed using SUPEX ECL reagent (Neuronex, Korea) and ImageQuant LAS-4000 (GE Healthcare, MA, USA) according to the manufacturer's instructions. Integrated blot densities were quantified using ImageJ.

As a result, as shown in FIGS. 2H to 2K, the anti-inflammatory activity of DTMB was confirmed to occur by reducing NF-κB, a major regulator of inflammatory responses within cells, and NLRP3, a component of inflammasome, was also confirmed to be reduced, confirming that DTMB regulates the inflammatory response of microglia.

In addition, to inhibit proteasomal degradation, cells were treated with MG132 (20 µM) for 6 hours and DTMB (25 µM) for 24 hours, and then the gene expression level of *NF-κB* was confirmed by qPCR analysis. As shown in the results of FIGS. 2L and 2M, this response was mediated by proteasomal degradation rather than transcriptional inhibition, since there was no change in the mRNA level of NF-κB.

### [Example 3]

### Effects of DTMB administration on memory and cognitive function improvement through Y maze test analysis in 5xFAD Alzheimer's dementia-induced mice

### 3-1. Preparation of experimental animals

5xFAD (Tg6799) mice were purchased from Jackson Laboratory (Bar Harbor, ME, USA), and male transgenic mice were mated with B6/SJL hybrid female mice. Genotyping was performed by PCR analysis of tail-cut samples. In this example, 8-week-old female mice were used for drug administration, and wild-type littermates were used as controls. All procedures related to animal behavior experiments were approved by the Institutional Animal Care and Use Committee of Pohang University of Science and Technology (POSTECH IACUC). All animal experiments were performed in accordance with the principles of the Animal Welfare Act, the PHS Animal Welfare Policy, and the NIH Guide for the Care and Use of Laboratory Animals. All mice were maintained under general conditions in the POSTECH animal facility according to institutional guidelines.

### 3-2. Drug treatment

To evaluate the effect of DTMB on 5xFAD mice, DTMB (10 mg/kg) and vehicle (1% carboxymethylcellulose and 10% DMSO) were orally administered to four groups (wild type, wild type + DTMB, 5xFAD, and 5xFAD + DTMB) 6 days per week for 3 months.

### 3-3. Y maze test

To confirm whether DTMB actually alleviates the pathology of Alzheimer's dementia, as shown in FIG. 3A, DTMB was orally administered to 5xFAD, an Alzheimer's dementia-induced mouse, at 10 mg/kg for 12 weeks starting at 8 weeks of age, and a Y maze test was performed the week after administration to examine the improvement of short-term memory and cognitive function. The measuring device consists of three branches, each of which is 42 cm long, 3 cm wide, and 12 cm high, and the angle at which the three branches folded is 120°. The device is made of black polyvinyl plastic, and the experiments were conducted after designating the three branches as A, B, and C, respectively. The experimental animal was placed, and 1 point (actual alternation) was awarded based on the number of times the animal's tail entered each branch and the number of times it entered each branch in turn for 8 minutes. Alternation behavior is defined as entering all three branches without overlapping and was calculated using the following mathematical formula. Alternation behavior(%) = (actual alternation/ maximum alternation) X 100 (maximum alternation: total number of entires)

First, as a result of checking the physical changes of the mice that completed the behavioral test, as shown in FIG. 3B, there was no change in body weight or tissue weight between the vehicle-treated mice and the DTMB-treated mice when DTMB was treated for 12 weeks (3 months).

As a result of the behavioral test, as shown in FIG. 3C, the alternation behavior of the 5xFAD mouse group decreased compared to the normal mouse group, but the alternation behavior increased statistically significantly with DTMB administration, confirming that short-term memory and cognitive function were improved. On the other hand, since the total branch entries, representing the total number of entries into each zone, showed no changes, it was confirmed that the spontaneous alternation was not due to changes in the activity levels of the mice.

### [Example 4]

### Effects of DTMB administration on memory and cognitive function improvement through Morris water maze test analysis in 5xFAD Alzheimer's dementia-induced mice

As shown in FIG. 3A, after oral administration of DTMB at 10 mg/kg, a water maze test was performed to examine memory improvement and cognitive function.

A platform with a diameter of 9 cm and a height of 25 cm was installed in one quadrant of a circular tank with a diameter of 90 cm and a height of 45 cm, and clean water at 20±2°C was filled about 1 cm above the platform, and the time it took for the experimental animal to find the platform was measured. Four signs were installed in the circular tank, dividing it into four quadrants, and the position of entry the water was changed, and the experiment was repeated four times a day for 60 seconds. If the experimental animal found the platform within 60 seconds, the experiment was completed. If the animal failed to find it, the location was guided by hand and the animal was made to stay for 10 seconds. After training to find a platform placed under the water surface of the tank for 5 days, on the last day of the experiment, the platform was removed and the time spent in the area where the platform was for 60 seconds was measured to measure learning ability.

As shown in FIG. 4A, the time taken by the 5xFAD mouse group to find the platform (Escape latency) increased compared to the normal mouse group through the 5-day training experiment, indicating that long-term memory was impaired. In addition, as shown in FIGS. 4B and 4C, the time spent by the 5xFAD mouse group in the target quadrant significantly decreased compared to the normal mouse group through the experiment on the last day, indicating that spatial perception was also impaired.

In contrast, the 5xFAD mouse group administered with DTMB showed similar levels of time spent finding the platform and time spent in the target quadrant to normal mice, as shown in the results of FIGS. 4A to 4C, confirming that long-term memory and spatial perception were improved to levels similar to normal mice. In addition, as confirmed in FIG. 4B, there was no difference in the swimming distance (Total path) between the normal mouse group administered the drug-free vehicle, the normal mouse group administered DTMB, the 5xFAD mouse group administered the drug-free vehicle, and the 5xFAD mouse group administered DTMB, indicating that there was no difference in the motor ability of the mice in each group.

Therefore, through the results of this example, it was possible to confirm the long-term memory improvement ability and cognitive function enhancement effect of DTMB.

### [Example 5]

### Confirmation of pathological improvement effect according to DTMB administration through analysis of Alzheimer's dementia mouse brain tissue

### Immunohistochemistry

In the case of Alzheimer's dementia, an increase in the formation of Aβ aggregates in brain tissue is observed as a common pathological phenomenon. Therefore, to determine whether DTMB administration inhibited the formation of Aβ aggregates, the amount of Aβ aggregates in the hippocampus and cerebral cortex, which are closely related to memory ability, was measured using immunochemical staining in 5xFAD Alzheimer's dementia-induced mice.

The mice were perfused with ice-cold phosphate-buffered saline (PBS). The brain tissue was used separately for molecular analysis or immunohistochemistry. The right hemisphere was fixed with 4% PFA and transferred to a sucrose solution (30%) for 24 hours. The right hemisphere was frozen in the optimal cutting temperature compound of dry ice. Coronal sections (18 µm thick) including the hippocampus were cut and prepared on slide glass. The tissue sections were then permeabilized and blocked with blocking solution (5% goat serum and 0.2% Triton X-100 in PBS) for 1 h at room temperature. Primary antibody incubation was performed overnight at 4 °C using anti-6e10 antibody (Biolegend, Japan, 1: 100). After washing, the sections were incubated with the appropriate secondary antibody (1:500) for 1 h at room temperature. The slides were counterstained with Hoechst and then fixed in fluorescent mounting medium (Dako, Denmark). All images were captured using an Axioplan2 microscope (Zeiss, Germany). Fluorescence was measured by calculating the % area and analyzed using ImageJ. The sample size was 10-15 mice per group.

As shown in FIGS. 5A and 5B, in the 5xFAD mouse group that was not administered DTMB (control in FIG. 5A; 5xFAD in FIG. 5B), the amount of Aβ aggregates in the hippocampus and cerebral cortex regions was significantly increased, but in the 5xFAD mouse group that was administered DTMB (DTMB in FIG. 5A; 5xFAD+DTMB in FIG. 5B), the amount of Aβ aggregates was significantly reduced.

### Western blot analysis

Aβ peptides are generated from amyloid precursor protein (APP) by the action of β- and γ-secretase. It is possible that DTMB acts on APP processing to inhibit the formation of Aβ aggregates. To investigate this possibility, the protein levels of total APP and components of β- and γ-secretase were measured in the hippocampus and cortex using western blot and qPCR.

To investigate the soluble and insoluble Aβ components, two extraction steps were performed. Left hemisphere samples were homogenized at a concentration of 100 mg/mL in cold Tris-buffered saline (TBS consisting of 50 mM Tris-pH 7.5 and 150 mM NaCl) with a protease inhibitor cocktail (Thermo Fisher Scientific, Waltham, MO, USA). After centrifugation at 15,000 rpm for 1 hour at 4 °C, the supernatant of the sample was used for western blotting. Insoluble proteins were extracted from the pellet by dissolving in 5 M guanidine buffer (5 M guanidine HCl and 150 mM NaCl-pH 7.5). Soluble and insoluble protein samples were stored at -80 °C.

Western blot analysis was performed in the same manner as in Example 2-4 using primary antibodies against nicastrin, pen2 (Cell Signaling Technology, Danvers, MA, USA), Aph1 (Invitrogen), amyloid C-terminal fragment (Santa Cruz Biotechnology, Dallas, TX, USA), and GAPDH (Bethyl Laboratories, Mongomery, TX, USA). Secondary antibodies from rabbit (Promega), rat, and goat (Bethyl Laboratories) were used, and detection was performed using SUPEX ECL reagent (Neuronex, Korea) and ImageQuant LAS-4000 (GE Healthcare, MA, USA) according to the manufacturer's instructions. Integrated blot densities were quantified using ImageJ.

As shown in FIGS. 5C to 5H, there were no changes in APP protein and mRNA levels or protein levels of β- and γ-secretase in the hippocampus, and the protein level of β-secretase was slightly decreased in the cortex of DTMB-treated mice.

### 5-3. Filter trap assay

The insoluble fraction of the protein lysate from 5xFAD mice was diluted in 1% SDS-PBS and boiled at 95 °C for 5 min. The membrane was pre-equilibrated with 1 X TBS. A dot blotter apparatus (Bio-Rad Laboratories, Hercules, CA, USA) was used for sample application, and the 6e10 antibody was used to detect the Aβ content in the brain lysate from 5xFAD mice treated with DTMB. The sample size was 3 mice per group.

As shown in FIGS. 5I and 5J, amyloid β monomers were not reduced by DTMB treatment, but Aβ aggregates in the insoluble fraction were reduced. These data indicate that DTMB reduces Aβ aggregates in the brain independent of the Aβ production process from APP.

### [Example 6]

### Analysis of the degree of gliosis due to hyperactivity of microglia and astrocytes in the brain tissue of Alzheimer's dementia mice according to DTMB administration

### 6-1. Immunohistochemistry

Since the anti-inflammatory effect of DTMB drug was observed at the cellular level, in order to confirm whether the inflammatory response was actually inhibited in the brain tissue according to drug administration, the brains of the 5xFAD Alzheimer's dementia-induced mice were extracted from the groups administered with DTMB and those not administered, and sections were made. The degree of gliosis was confirmed through immunochemical analysis using antibodies against IBA-1, a specific protein of microglia, and GFAP, a specific protein of astrocytes. Specifically, immunohistochemistry was performed in the same manner as in Example 6-1 using anti-Iba1 antibody (Wako, Japan, 1:200) and anti-GFAP antibody (Abcam, Cambridge, UK, 1:200) as primary antibodies.

As confirmed in FIGS. 6A to 6F, when observing the cerebral cortex (FIGS. 6A, 6C, 6E, and 6F) and hippocampus (FIGS. 6B, 6D, 6E, and 6F) tissues of the brain, the 5xFAD mouse group showed a significant increase in the hyperactivity of microglia and astrocytes compared to the normal mouse group, but in the mouse group administered DTMB, the hyperactivity of microglia and astrocytes, i.e., gliosis, was significantly reduced.

### 6-2. Real-time quantitative PCR (qPCR)

The gene expression levels of proinflammatory cytokines and related enzymes (Il-1β, Il-6, and iNOS) in the brain tissues of the DTMB-administered and non-administered groups of 5xFAD Alzheimer's dementia-induced mice were confirmed by qPCR. As a result, as shown in FIG. 8G, the expression levels of proinflammatory cytokines and related enzymes (Il-1β, Il-6, and iNOS) were also decreased in the DTMB-treated transgenic mice.

### Western blot

The protein levels of NLRP3, NF-κB, and ASC in the hippocampal tissues isolated from 5xFAD mice and DTMB-treated 5xFAD mice were confirmed by Western blot. Western blot analysis was performed in the same manner as in Example 2-4 using primary antibodies against NLRP3, NF-κB, and ASC (Cell Signaling Technology, Danvers, MA, USA).

As shown in FIGS. 6H and 6I, the protein levels of NLRP3 and NF-κB were decreased in the hippocampal brain lysate, but the ASC level did not change significantly. These results suggest that DTMB reduces chronic inflammatory pathology in Alzheimer's disease mouse model.

### [Example 7]

### Functional changes in microglia isolated from mice administered DTMB

To elucidate whether the gliosis reduction effect of DTMB administration confirmed in Example 6 was due to functional changes in microglia, brain tissues from mice administered DTMB were extracted, microglia were isolated, and the expression of genes related to inflammation and activation in microglia was analyzed to what extent they changed.

To isolate adult microglia from the brains of 5xFAD mice treated with DTMB or vehicle, a magnetic-activated cell sorting (MACS) system was used. Whole brain tissue was dissociated to the single-cell level using the gentleMACs dissociation kit (Miltenyi Biotech, Bergisch Gladbach, Germany) according to the manufacturer's instructions. To isolate the immune cell fraction, a 47% Percoll concentration gradient was applied to the single-cell mixture and centrifuged at 2,000 rpm for 10 min. Adult microglia were isolated by LS column after incubation with anti-CD11b MicroBeads (Miltenyi Biotech). RT-PCR was performed using the isolated adult microglia to analyze the expression of microglial activation and inflammation-related genes. The sample size was 3 mice per group.

As shown in FIG. 7, the gene expression of cytokine genes *TNF-α, IL-6,* and *IL-1β,* chemokine genes *Cxcl10* and *clec7a,* and microglial activity-related genes *Iba-1, Trem2,* and *Itgax2* increased in microglial cells of the 5xFAD mouse group compared to the normal mouse group, but the expression levels of all of these genes decreased in microglial cells of the 5xFAD mouse group administered with DTMB, confirming that DTMB can suppress excessive microglial activity.

### Statistical analysis

All statistical analyses were performed using GraphPad Prism version 9.2. Normality tests were performed before statistical analysis using the GraphPad system. Comparisons between two groups were analyzed by two-tailed unpaired Student's t test. For comparisons between more than two groups, one-way or two-way analysis of variance (ANOVA) with Tukey's test was used. A p-value less than 0.05 was considered statistically significant. All quantitative data are presented as mean ± standard error of the mean (SEM).

## Claims

1. A pharmaceutical composition for preventing or treating degenerative brain diseases, comprising a compound represented by Chemical Formula 1 below, a stereoisomer thereof or a pharmaceutically acceptable salt thereof as an active ingredient:

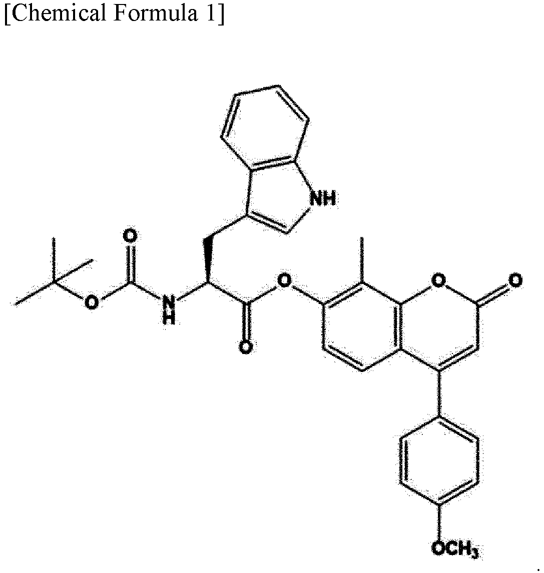

2. The pharmaceutical composition of claim 1,
wherein the stereoisomer comprises a racemate, an enantiomer, a diastereomer, a mixture of enantiomers or a mixture of diastereomers.

3. The pharmaceutical composition of claim 1,
wherein the degenerative brain disease comprises one or more selected from the group consisting of Dementia, Autism, Alzheimer's disease, Huntington's disease, Vascular dementia, Stroke, Ischemic stroke, Traumatic brain injury, Amnesia, Parkinson's disease, Pick disease, Creutzfeldt-Jakob disease, Peripheral neuropathy, Amyotrophic lateral sclerosis, multiple sclerosis and mild cognitive impairment.

4. The pharmaceutical composition of claim 1,
wherein the compound, the stereoisomer thereof or the pharmaceutically acceptable salt thereof activates all of PPARα, PPARβ/δ and PPARγ.

5. The pharmaceutical composition of claim 1,
wherein the compound, the stereoisomer thereof or the pharmaceutically acceptable salt thereof exhibits one or more activities selected from the following i) to iii):
i) inhibiting inflammatory responses by activating PPAR transcription factors in microglia;
ii) inhibiting the formation of Aβ aggregates in brain tissue; and
iii) inhibiting gliosis caused by excessive activity of microglia and astrocytes.

6. A health functional food composition for preventing or ameliorating degenerative brain diseases, comprising a compound represented by Chemical Formula 1 below, a stereoisomer thereof or a sitologically salt thereof as an active ingredient:

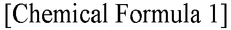

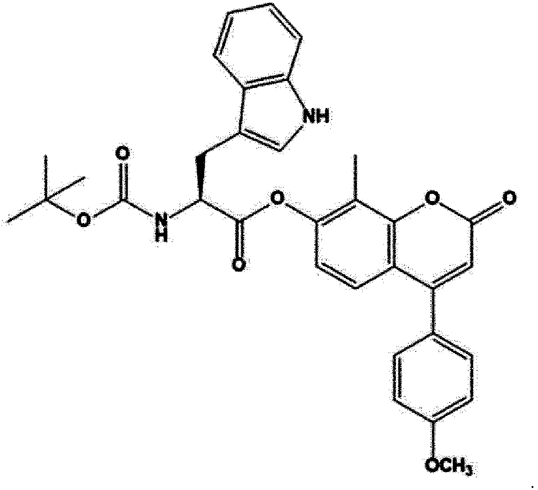

7. The health functional food composition of claim 6,
wherein the stereoisomer comprises a racemate, an enantiomer, a diastereomer, a mixture of enantiomers or a mixture of diastereomers.

8. The health functional food composition of claim 6,
wherein the degenerative brain disease comprises one or more selected from the group consisting of Dementia, Autism, Alzheimer's disease, Huntington's disease, Vascular dementia, Stroke, Ischemic stroke, Traumatic brain injury, Amnesia, Parkinson's disease, Pick disease, Creutzfeldt-Jakob disease, Peripheral neuropathy, Amyotrophic lateral sclerosis, multiple sclerosis and mild cognitive impairment.

9. The health functional food composition of claim 6,
wherein the compound, the stereoisomer thereof or the sitologically salt thereof activates all of PPARα, PPARβ/δ and PPARγ.

10. The health functional food composition of claim 6,
wherein the compound, the stereoisomer thereof or the sitologically salt thereof exhibits one or more activities selected from the following i) to iii):
i) inhibiting inflammatory responses by activating PPAR transcription factors in microglia;
ii) inhibiting the formation of Aβ aggregates in brain tissue; and
iii) inhibiting gliosis caused by excessive activity of microglia and astrocytes.

11. A composition for enhancing learning ability, improving memory or ameliorating cognitive function, comprising a compound represented by Chemical Formula 1 below, a stereoisomer thereof or a pharmaceutically or sitologically salt thereof as an active ingredient:

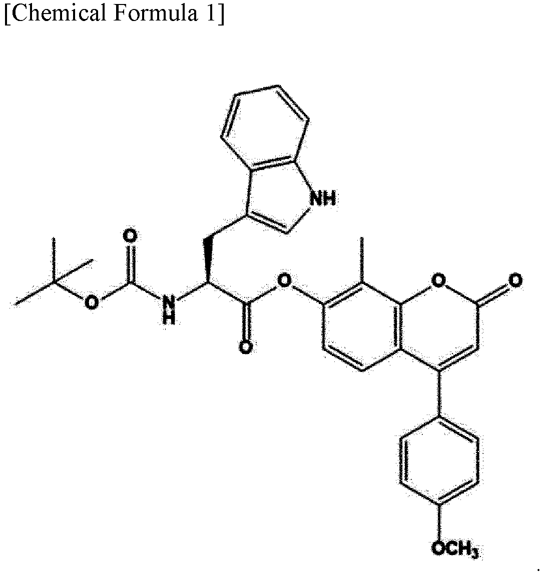

12. The composition of claim 11,
wherein the stereoisomer comprises a racemate, an enantiomer, a diastereomer, a mixture of enantiomers or a mixture of diastereomers.

13. The composition of claim 11,
wherein the compound, the stereoisomer thereof or the pharmaceutically or sitologically salt thereof activates all of PPARα, PPARβ/δ and PPARγ.

14. The composition of claim 11,
wherein the compound, the stereoisomer thereof or the pharmaceutically or sitologically salt thereof exhibits one or more activities selected from the following i) to iii):
i) inhibiting inflammatory responses by activating PPAR transcription factors in microglia;
ii) inhibiting the formation of Aβ aggregates in brain tissue; and
iii) inhibiting gliosis caused by excessive activity of microglia and astrocytes.

15. The composition of claim 11,
wherein the composition is pharmaceutical or health functional food composition.

16. A method for preventing, ameliorating, or treating degenerative brain diseases, comprising administering a compound represented by Chemical Formula 1 below, a stereoisomer thereof or a pharmaceutically or sitologically salt thereof to a subject in need thereof:

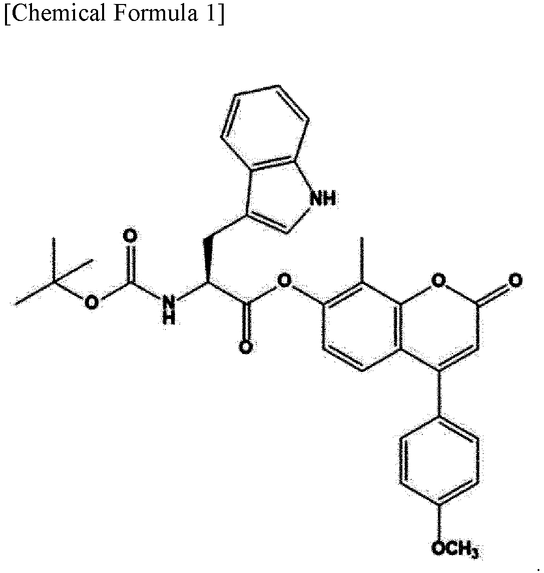

17. The method of claim 16,
wherein the stereoisomer comprises a racemate, an enantiomer, a diastereomer, a mixture of enantiomers or a mixture of diastereomers.

18. The method of claim 16,
wherein the degenerative brain disease comprises one or more selected from the group consisting of Dementia, Autism, Alzheimer's disease, Huntington's disease, Vascular dementia, Stroke, Ischemic stroke, Traumatic brain injury, Amnesia, Parkinson's disease, Pick disease, Creutzfeldt-Jakob disease, Peripheral neuropathy, Amyotrophic lateral sclerosis, multiple sclerosis and mild cognitive impairment.

19. The method of claim 16,
wherein the compound, the stereoisomer thereof or the pharmaceutically or sitologically salt thereof activates all of PPARα, PPARβ/δ and PPARγ.

20. The method of claim 16,
wherein the compound, the stereoisomer thereof or the pharmaceutically or sitologically salt thereof exhibits one or more activities selected from the following i) to iii):
i) inhibiting inflammatory responses by activating PPAR transcription factors in microglia;
ii) inhibiting the formation of Aβ aggregates in brain tissue; and
iii) inhibiting gliosis caused by excessive activity of microglia and astrocytes.

21. A method for enhancing learning ability, improving memory or ameliorating cognitive decline, comprising administering a compound represented by Chemical Formula 1 below, a stereoisomer thereof or a pharmaceutically or sitologically salt thereof to a subject in need thereof:

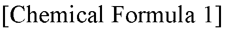

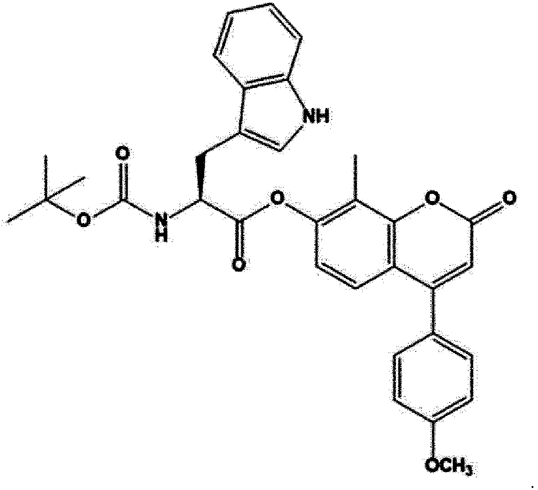

22. A use of a compound represented by Chemical Formula 1 below, a stereoisomer thereof or a pharmaceutically or sitologically salt thereof for the manufacture of a medicament for preventing, ameliorating, or treating degenerative brain diseases:

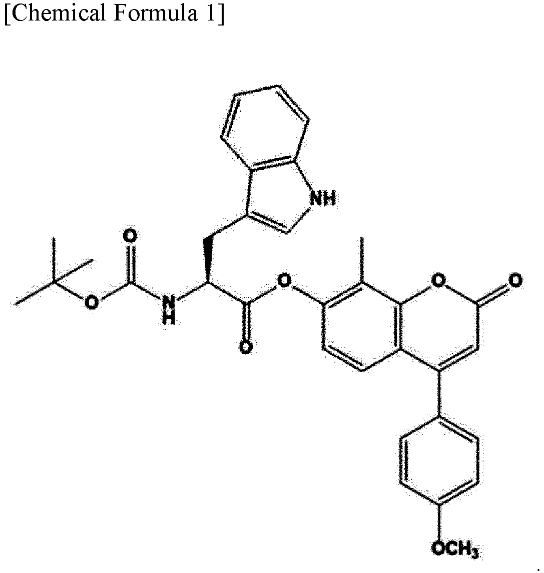

23. The use of claim 22,
wherein the stereoisomer comprises a racemate, an enantiomer, a diastereomer, a mixture of enantiomers or a mixture of diastereomers.

24. The use of claim 22,
wherein the degenerative brain disease comprises one or more selected from the group consisting of Dementia, Autism, Alzheimer's disease, Huntington's disease, Vascular dementia, Stroke, Ischemic stroke, Traumatic brain injury, Amnesia, Parkinson's disease, Pick disease, Creutzfeldt-Jakob disease, Peripheral neuropathy, Amyotrophic lateral sclerosis, multiple sclerosis and mild cognitive impairment.

25. The use of claim 22,
wherein the compound, the stereoisomer thereof or the pharmaceutically or sitologically salt thereof activates all of PPARα, PPARβ/δ and PPARγ.

26. The use of claim 22,
wherein the compound, the stereoisomer thereof or the pharmaceutically or sitologically salt thereof exhibits one or more activities selected from the following i) to iii):
i) inhibiting inflammatory responses by activating PPAR transcription factors in microglia;
ii) inhibiting the formation of Aβ aggregates in brain tissue; and
iii) inhibiting gliosis caused by excessive activity of microglia and astrocytes.

27. A use of a compound represented by Chemical Formula 1 below, stereoisomers thereof or pharmaceutically or sitologically salts thereof for the manufacture of a medicament for enhancing learning ability, improving memory or ameliorating cognitive decline:
